# EUROPEAN PATENT SPECIFICATION

(11) **EP 2 046 343 B1**
(45) Date of publication and mention of the grant of the patent: **14.05.2014**
(21) Application number: 07873711.1
(22) Date of filing: 27.07.2007
(51) Int. Cl.: A61K 31/407, A61P 39/00, A61K 47/16, A61K 47/32, A61K 47/34, A61K 47/40, A61K 9/00

(54) **FORMULATIONS OF RADIOPROTECTIVE ALPHA, BETA UNSATURATED ARYL SULFONES**
FORMULIERUNGEN AUS STRAHLENSCHÜTZENDEN UNGESÄTTIGTEN ALPHA- UND BETA-ARYLSULFONEN
FORMULATIONS D'ARYLSULFONES ALPHA, BETA -INSATURÉES RADIOPROTECTRICES

(30) Priority: 28.07.2006 US 833842 P
(43) Date of publication of application: 15.04.2009
(73) Proprietor: Onconova Therapeutics, Inc., Newtown, PA 18940 (US)
(72) Inventor: MANIAR, Manoj, Fremont, CA 94539 (US); BELL, Stanley, C., Narbeth, PA 19072 (US)
(74) Representative: Howard, Paul Nicholas
(86) International application number: PCT/US2007/016879
(87) International publication number: WO 2008/105808

(56) References cited:
- WO-A-2006/010152
- WO-A-2007/016201
- US-B2- 6 667 346
- US-B2- 6 683 093
- DASH S K ET AL.: "Preformulation development of a parenteral formulation for ON 01210.Na, a radioprotectant" AAPS ANNUAL MEETING AND EXPOSITION, [Online] 5 November 2005 (2005-11-05), - 10 November 2005 (2005-11-10) pages 1-1, XP002549966 Retrieved from the Internet: URL:http://abstracts.aapspharmaceutica.com /ExpoAAPS05/CC/forms/attendee/index.aspx?c ontent=sessionInfo&sessionId=577> [retrieved on 2009-10-05]
- ALFIERI A A ET AL: "Radiation damage protection by the benzyl styrl sulfone analog, Ex-Rad" INTERNATIONAL JOURNAL OF RADIATION ONCOLOGY BIOLOGY PHYSICS, vol. 60, no. 1, Suppl. S, 2004, pages S367-S368, XP002549965 & 46TH ANNUAL MEETING OF THE AMERICAN-SOCIETY-FOR-THERAPEUTIC-RADIOLOGY AND-ONCOLOGY; ATLANTA, GA, USA; OCTOBER 03 -07, 2004 ISSN: 0360-3016

## Description

### FIELD OF THE INVENTION

The invention relates to solution formulations for effective pharmacological delivery of cytoprotective agents, e.g., at least one α, β unsaturated aryl sulfone, for the protection and/or treatment of cells and tissues from/of the toxicity of ionizing radiation.

### BACKGROUND OF THE INVENTION

While anti-radiation suits or other protective gear may be effective at reducing radiation exposure, such gear is expensive, unwieldy, and generally not available to public. Moreover, radioprotective gear will not protect normal tissue adjacent a tumor from stray radiation exposure during radiotherapy. Pharmaceutical radioprotectants offer a cost-efficient, effective and easily available alternative to radioprotective gear. However, previous attempts at radioprotection of normal cells with pharmaceutical compositions have not been entirely successful. For example, cytokines directed at mobilizing the peripheral blood progenitor cells confer a myeloprotective effect when given prior to radiation (Neta et al., Semin. Radiat. Oncol. 6:306-320, 1996), but do not confer systemic protection. Other chemical radioprotectors administered alone or in combination with biologic response modifiers have shown minor protective effects in mice, but application of these compounds to large mammals was less successful, and it was questioned whether chemical radioprotection was of any value (Maisin, J.R., Bacq and Alexander Award Lecture. "Chemical radioprotection: past, present, and future prospects", Int J. Radiat Biol. 73:443-50, 1998). Pharmaceutical radiation sensitizers, which are known to preferentially enhance the effects of radiation in cancerous tissues, are clearly unsuited for the general systemic protection of normal tissues from exposure to ionizing radiation.

WO2007/016201 and US-6,667,346 describe radioprotective α,β -unsaturated aryl sulfones.

What is needed, therefore, is a practical means to protect subjects from the toxicity of radiation, wherein the subjects are either scheduled to incur or are at risk for incurring, or have incurred, exposure to ionizing radiation.

### SUMMARY OF THE INVENTION

Exemplary pharmaceutical compositions of the present invention are defined according to the claims.

### BRIEF DESCRIPTION OF THE FIGURES

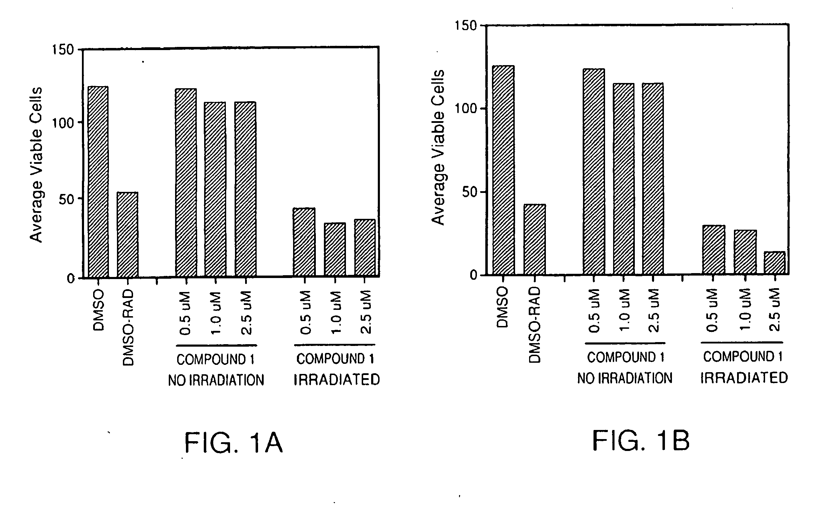

FIG.1A and 1B show the effect of 5 Gy and 10 Gy of ionizing radiation, respectively, on the viability of DU145 prostate tumor cells in the presence or absence of (E)-4-fluorostyryl-4-chlorobenzylsulfone.

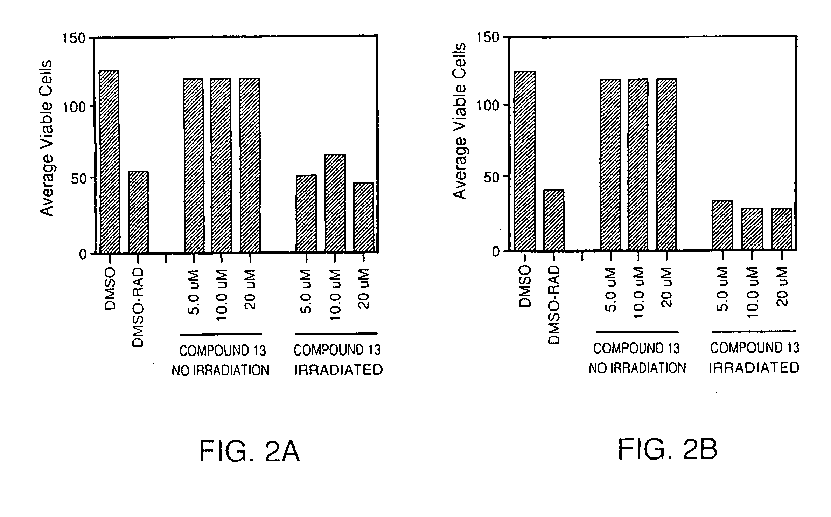

FIG.2A and 2B show the effect of 5 Gy and 10 Gy of ionizing radiation, respectively, on the viability of DU145 prostate tumor cells in the presence or absence of (E)-4-carboxystyryl-4-chlorobenzylsulfone.

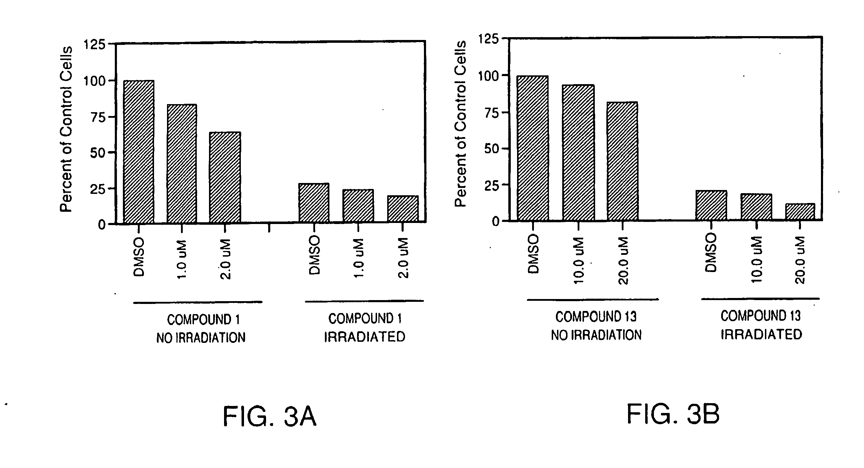

FIG.3A and 3B show the effect of 10 Gy ionizing radiation on the viability of DU145 prostate tumor cells treated post-irradiation, respectively, with (E)-4-fluorostyryl-4-chlorobenzylsulfone and (E)-4-carboxystyryl-4-chlorobenzylsulfone.

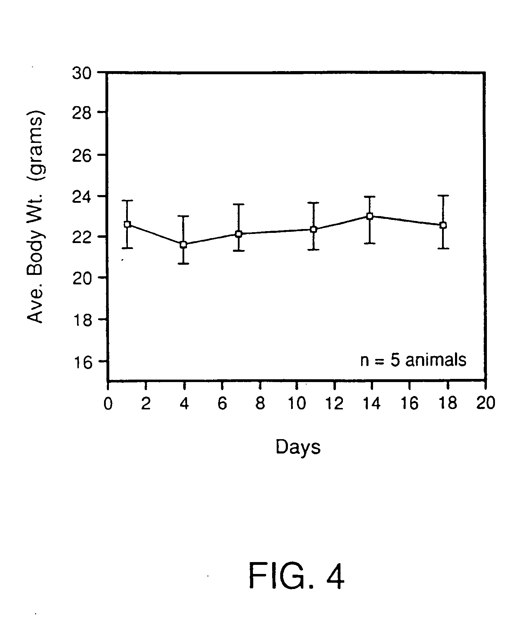

FIG.4 is a plot of average body weight (grams) vs. time(days) for C57B6/J mice given 4 mg/kg (E)-4-fluorostyryl-4-chlorobenzylsulfone every other day for 18 days.
FIG.5 is a Kaplan Meyer survival plot of C57B6/J mice pre-treated with (E)-4-carboxystyryl-4-chlorobenzylsulfone at 18 and 6 hrs before receiving 8 Gy of ionizing radiation.
FIG.6 is a Kaplan Meyer survival plot of C57B6/J mice treated with (E)-4-carboxystyryl-4-chlorobenzylsulfone after receiving 8 Gy of ionizing radiation.

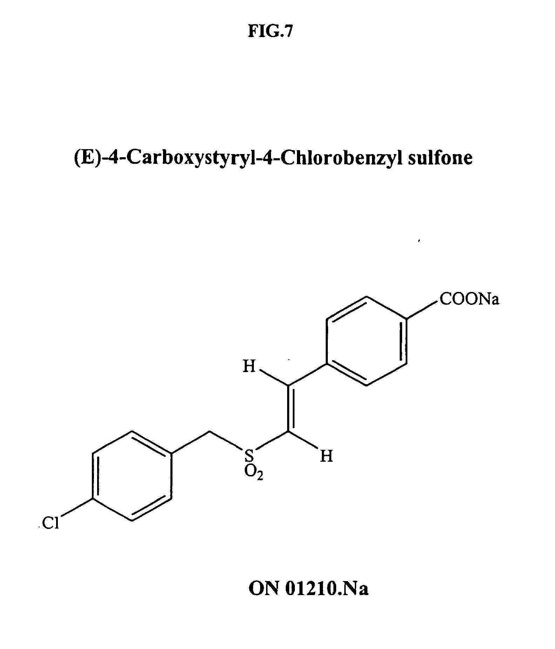

FIG.7 shows the structure of the sodium salt of 4-chlorobenzyl-4-carboxystyryl sulfone (ON.1210.Na).

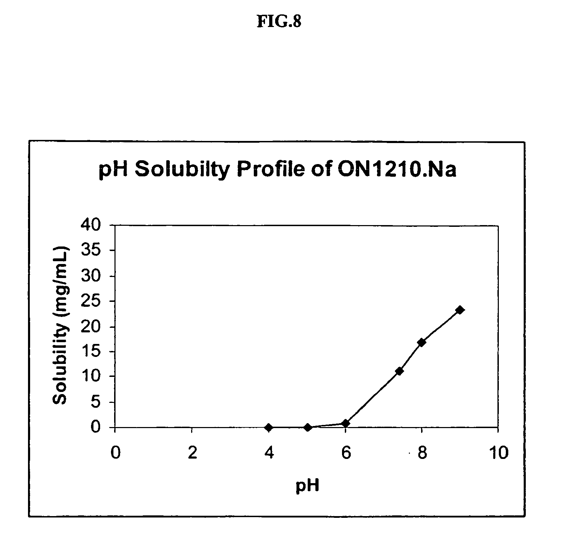

FIG.8 shows pH solubility profiles of ON.1210.Na at ambient temperature.

### DETAILED DESCRIPTION OF THE INVENTION

Unless defined otherwise, all technical and scientific terms used herein have the same meaning as is commonly understood by one of skill in the art to which this invention belongs. The entireties of the disclosures of U.S. Patent Nos. 6,656,973 and 6,667,346 are particularly noted.

The major biological effects of radiation exposure are the destruction of bone marrow cells, gastrointestinal (GI) damage, lung pneumonitis, and central nervous system (CNS) damage. The long-term effects of radiation exposure include an increase in cancer rates. It has been estimated that the exposure of 100 rems (roentgen equivalent man: a measurement used to quantify the amount of radiation that would produce harmful biological effects) would produce ARS symptoms. Exposure levels above 300 rems would result in the death of approximately 50% of the exposed population.

The α, β-unsaturated aryl sulfones, in particular benzyl styryl sulfones, provide significant and selective systemic protection of normal cells from radiation-induced damage in animals. When used in radiotherapy techniques, these compounds also exhibit independent toxicity to cancer cells. Compositions and formulations of α,β unsaturated aryl sulfones described herein protect normal cells and tissues from the effects of acute and chronic exposure to ionizing radiation. The α,β unsaturated aryl sulfones are also operationally cytotoxic in tumor cells. Compositions described herein are intended for prophylactic use, for example, to enhance survival in personnel who are in imminent danger of exposure to life-threatening levels of x-ray or gamma radiation, and/or to enhance survival in personnel who have just received life-threatening levels of xray or gamma radiation. In animal efficacy studies, pre-treatment with ON.01210.Na, for example, by the intravenous, sub-cutaneous or intraperitoneal route resulted in protection of mice from a lethal dose of ionizing radiation.

Subjects may be exposed to ionizing radiation when undergoing therapeutic irradiation for the treatment of proliferative disorders. Such disorders include cancerous and non-cancer proliferative disorders. Formulations described herein are effective in protecting normal cells during therapeutic irradiation of a broad range of tumor types, including but not limited to the following: breast, prostate, ovarian, lung, colorectal, brain (i.e., glioma) and renal. The compositions are also effective against leukemic cells, for example. The compositions are useful in protecting normal cells during therapeutic irradiation of abnormal tissues in non-cancer proliferative disorders, including but not limited to hemangiomatosis in new born, secondary progressive multiple sclerosis, chronic progressive myelodegenerative disease, neurofibromatosis, ganglioneuromatosis, keloid formation, Paget's Disease of the bone, fibrocystic disease of the breast, Peronies and Duputren's fibrosis, restenosis and cirrhosis.

Therapeutic ionizing radiation may be administered to a subject on any schedule and in any dose consistent with the prescribed course of treatment, as long as the α,β unsaturated aryl sulfone radioprotectant compound is administered prior to the radiation. The course of treatment differs from subject to subject, and those of ordinary skill in the art can readily determine the appropriate dose and schedule of therapeutic radiation in a given clinical situation. Compositions of α,β unsaturated aryl sulfones described herein should be administered far enough in advance of the therapeutic radiation such that the compound is able to reach the normal cells of the subject in sufficient concentration to exert a radioprotective effect on the normal cells. At least one α,β unsaturated aryl sulfone may be administered as much as about 24 hours, preferably no more than about 18 hours, prior to administration of the radiation. In one embodiment, an α,β unsaturated aryl sulfone formulation is administered at least about 6 - 12 hours before administration of the therapeutic radiation. Most preferably, the α,β unsaturated aryl sulfone is administered once at about 18 hours and again at about 6 hours before the radiation exposure. One or more α,β unsaturated aryl sulfones may be administered simultaneously, or different α,β unsaturated aryl sulfones may be administered at different times during the treatment.

Preferably, an about 24 hour period separates administration of α,β unsaturated aryl sulfone and the therapeutic radiation. More preferably, the administration of α,β unsaturated aryl sulfone and the therapeutic radiation is separated by about 6 to 18 hours. This strategy will yield significant reduction in radiation-induced side effects without affecting the anticancer activity of the therapeutic radiation.

An acute dose of ionizing radiation which may cause remediable radiation damage includes a localized or whole body dose, for example, between about 10,000 millirem (0.1 Gy) and about 1,000,000 millirem (10 Gy) in 24 hours or less, preferably between about 25,000 millirem (0.25 Gy) and about 200,000 (2 Gy) in 24 hours or less, and more preferably between about 100,000 millirem (1 Gy) and about 150,000 millirem (1.5 Gy) in 24 hours or less.

A chronic dose of ionizing radiation which may cause remediable radiation damage includes a whole body dose of about 100 millirem (.001 Gy) to about 10,000 millirem (0.1 Gy), preferably a dose between about 1000 millirem (.01 Gy) and about 5000 millirem (.05 Gy) over a period greater than 24 hours, or a localized dose of 15,000 millirem (0.15 Gy) to 50,000 millirem (0.5 Gy) over a period greater than 24 hours.

In the event of a terrorist attack releasing lethal amounts of radiation, radioprotective compositions described herein should provide protection when administered just *after,* e.g., up to about four hours after, exposure.

### I. EXAMPLE STRUCTURAL GENUS

By "α,β unsaturated aryl sulfone" as used herein is meant a chemical compound containing one or more α,β unsaturated aryl sulfone groups: 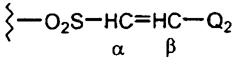 wherein Q₂ is substituted or unsubstituted aryl, and the hydrogen atoms attached to the α and β carbons are optionally replaced by other chemical groups.

By "substituted" means that an atom or group of atoms has replaced hydrogen as the substituent attached to a ring atom. The degree of substitution in a ring system may be mono-, di-, tri- or higher substitution.

The term "aryl" employed alone or in combination with other terms, means, unless otherwise stated, a carbocyclic aromatic system containing one or more rings (typically one, two or three rings) wherein such rings may be attached together in a pendent manner or may be fused. Examples include phenyl; anthracyl; and naphthyl, particularly 1-naphthyl and 2-naphthyl. It is understood that the term "aryl" is not limited to ring systems with six members.

The term "heteroaryl" by itself or as part of another substituent means, unless otherwise stated, an unsubstituted or substituted, stable, mono- or multicyclic heterocyclic aromatic ring system which consists of carbon atoms and from one to four heteroatoms selected from the group consisting ofN, O, and S, and wherein the nitrogen and sulfur heteroatoms may be optionally oxidized, and the nitrogen atom may be optionally quatemized. The heterocyclic system may be attached, unless otherwise stated, at any heteroatom or carbon atom which affords a stable structure.

Examples of such heteroaryls include benzimidazolyl, particularly 2-benzimidazolyl; benzofuryl, particularly 3-, 4-, 5-, 6- and 7-benzofuryl; 2-benzothiazolyl and 5-benzothiazolyl; benzothienyl, particularly 3-, 4-, 5-, 6-, and 7-benzothienyl; 4-(2-benzyloxazolyl); furyl, particularly 2- and 3-furyl; isoquinolyl, particularly 1- and 5-isoquinolyl; isoxazolyl, particularly 3-, 4- and 5-isoxazolyl; imidazolyl, particularly 2-, -4 and 5-imidazolyl; indolyl, particularly 3-, 4-, 5-, 6- and 7-indolyl; oxazolyl, particularly 2-, 4- and 5-oxazolyl; purinyl; pyrrolyl, particularly 2-pyrrolyl, 3-pyrrolyl; pyrazolyl, particularly 3- and 5-pyrazolyl; pyrazinyl, particularly 2-pyrazinyl; pyridazinyl, particularly 3- and 4-pyridazinyl; pyridyl, particularly 2-, 3- and 4-pyridyl; pyrimidinyl, particularly 2- and 4-pyrimidyl; quinoxalinyl, particularly 2- and 5-quinoxalinyl; quinolinyl, particularly 2- and 3-quinolinyl; 5-tetrazolyl; 2-thiazolyl; particularly 2-thiazolyl, 4-thiazolyl and 5-thiazolyl; thienyl, particularly 2- and 3-thienyl; and 3-(1,2,4-triazolyl).

According to one alternative, the α,β unsaturated aryl sulfone group is a styryl sulfone group: 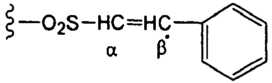 wherein the hydrogen atoms attached to the α and β carbons are optionally replaced by other chemical groups, and the phenyl ring is optionally substituted.

By "styryl sulfone" or "styryl sulfone compound" or "styryl sulfone therapeutic" as used herein is meant a chemical compound containing one or more such styryl sulfone groups.

The α,β unsaturated aryl sulfone radioprotective compounds are characterized by cis-trans isomerism resulting from the presence of a double bond. Stearic relations around a double bond are designated as "Z" or "E". Both configurations are included in the scope of "α,β unsaturated aryl sulfone":

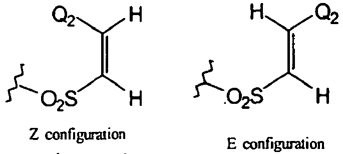

According to one alternative, the α,β unsaturated aryl sulfone compound is a compound of the formula I: 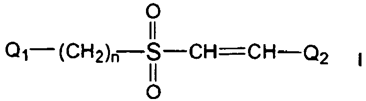 wherein:
n is one or zero;
Q₁ and Q₂ are, same or different, are substituted or unsubstituted aryl, or substituted or unsubstituted heteroaryl.

Preferably, n in formula I is one, that is, the compounds comprise α,β unsaturated benzylsulfones, e.g. styryl benzylsulfones.

In one alternative according to formula I, Q₁ and/or Q₂ are selected from substituted and unsubstituted heteroaryl; for example, (E)-3-furanethenyl-2,4-dichlorobenzylsulfone.

In another alternative according to formula 1, Q₁ and Q₂ are selected from substituted and unsubstituted phenyl.

More specific compounds where Q₁ and Q₂ are selected from substituted and unsubstituted phenyl comprise compounds of the formula II: 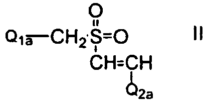 wherein:
Q₁ₐ and Q₂ₐ are independently selected from the group consisting of phenyl and mono-, di-, tri-, tetra- and penta-substituted phenyl where the substituents, which may be the same or different, are independently selected from the group consisting of hydrogen, halogen, C1-C8 alkyl, C1-C8 alkoxy, nitro, cyano, carboxy, hydroxy, phosphonato, amino, sulfamyl, acetoxy, dimethylamino(C2-C6 alkoxy), C1-C6 trifluoroalkoxy and trifluoromethyl.

In one alternative, compounds of formula II are at least di-substituted on at least one ring, that is, at least two substituents on at least one ring are other than hydrogen. In another embodiment, compounds of formula II are at least trisubstituted on at least one ring, that is, at least three substituents on at least one ring are other than hydrogen.

In one alternative, the radioprotective compound has the formula III: 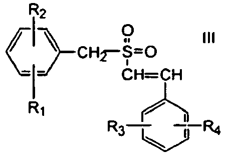 wherein R₁, R₂, R₃ and R₄ are independently selected from the group consisting of hydrogen, halogen, C1-C8 alkyl, C1-C8 alkoxy, nitro, cyano, carboxy, hydroxy phosphonato, amino, sulfamyl, acetoxy, dimethylamino(C2-C6 alkoxy), C1-C6 trifluoroalkoxy and trifluoromethyl.

According to another alternative, the radioprotective compound is according to formula III, and R₁ and R₂ are independently selected from the group consisting of hydrogen, halogen, cyano, and trifluoromethyl; and R₃ and R₄ are independently selected from the group consisting of hydrogen and halogen.

According to one sub-group of formula III, the radioprotective α,β unsaturated aryl sulfone compound is a compound of the formula IIIa, wherein R₂ and R₄ are other than hydrogen: 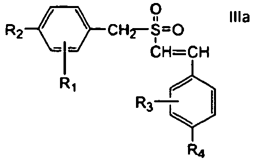 Compounds according to formula IIIa having the E-configuration include (E)-4-fluorostyryl-4-chlorobenzylsulfone; (E)-4-chlorostyryl-4-chlorobenzylsulfone; (E)-2-chloro-4-fluorostyryl-4-chlorobenzylsulfone; (E)-4-carboxystyryl-4-chlorobenzyl sulfone; (E)-4-fluorostyryl-2,4-dichlorobenzylsulfone; (E)-4-fluorostyryl-4-bromobenzylsulfone; (E)-4-chlorostyryl-4-bromobenzylsulfone; (E)-4-bromostyryl-4-chlorobenzylsulfone; (E)-4-fluorostyryl-4-trifluoromethylbenzylsulfone; (E)-4-fluorostyryl-3,4-dichlorobenzylsulfone; (E)-4-fluorostyryl-4-cyanobenzylsulfone; (E)-2,4-dichloro-4-chlorobenzylsulfone; (E)-4-fluorostyryl-4-chlorophenylsulfone and (E)-4-chlorostyryl-2,4-dichlorobenzylsulfone.

According to another alternative, compounds of formula IIIa have the Z configuration wherein R₁ and R₃ are hydrogen, and R₂ and R₄ are selected from the group consisting of 4-halogen. Such compounds include, for example, (Z)-4-chlorostyryl-4-chlorobenzylsulfone; (Z)-4-chlorostyryl-4-fluorobenzylsulfone; (Z)-4-fluorostyryl-4-chlorobenzylsulfone; (Z)-4-bromostyryl-4-chlorobenzylsulfone; and (Z)-4-bromostyryl-4-fluorobenzylsulfone.

According to another alternative, the radioprotective α,β unsaturated aryl sulfone compound is a compound of the formula IV: 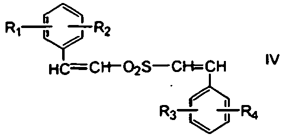 wherein
R₁, R₂, R₃, and R₄ are independently selected from the group consisting of hydrogen, halogen, C1-8 alkyl, C1-8 alkoxy, nitro, cyano, carboxy, hydroxy, and trifluoromethyl.

In one alternative, R₁ in formula IV is selected from the group consisting of hydrogen, chlorine, fluorine and bromine; and R₂, R₃ and R₄ are hydrogen. A preferred compound of formula IV is (Z)-styryl-(E)-2-methoxy-4-ethoxystyrylsulfone.

According to yet another alternative, the radioprotective α,β unsaturated aryl sulfone compound is a compound of the formula V:
wherein 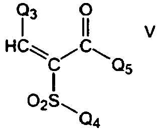
Q₃, Q₄ and Q₅ are independently selected from the group consisting of phenyl and mono-, di-, tri-, tetra- and penta-substituted phenyl where the substituents, which may be the same or different, are independently selected from the group consisting of halogen, C1-C8 alkyl, C1-C8 alkoxy, nitro, cyano, carboxy, hydroxy, phosphonato, amino, sulfamyl, acetoxy, dimethylamino(C2-C6 alkoxy), C1-C6 trifluoroalkoxy and trifluoromethyl.

According to one sub-group of formula V, the radioprotective α,β unsaturated aryl sulfone compound is a compound of the formula Va: wherein 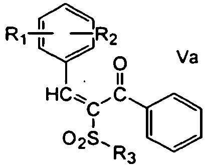
R₁ and R₂ are independently selected from the group consisting of hydrogen, halogen, C1-C8 alkyl, C1-8 alkoxy, nitro, cyano, carboxyl, hydroxyl, and trifluoromethyl; and
R₃ is selected from the group consisting of unsubstituted phenyl, monosubstituted phenyl and di-substituted phenyl, the substituents on the phenyl ring being independently selected from the group consisting of halogen and C1-8 alkyl.

More specifically R₁ in formula Va is selected from the group consisting of fluorine and bromine; R₂ is hydrogen; and R₃ is selected from the group consisting of 2-chlorophenyl, 4-chlorophenyl, 4-fluorophenyl, and 2-nitrophenyl.

A specific radioprotective styryl sulfone according to formula Va is the compound wherein R₁ is fluorine, R₂ is hydrogen and R₃ is phenyl, that is, the compound 2-(phenylsulfonyl)-1-phenyl-3-(4-fluorophenyl)-2-propen-1-one.

By "dimethylamino(C2-C6 alkoxy)" is meant (CH₃)₂N(CH₂)ₙO- wherein n is from 2 to 6. More specifically, n is 2 or 3. Most specifically, n is 2, that is, the group is the dimethylaminoethoxy group, that is, (CH₃)₂NCH₂CH₂O-.

By "phosphonato" is meant the group -PO(OH)₂.

By "sulfamyl" is meant the group -SO₂NH₂.

Where a substituent on an aryl nucleus is an alkoxy group, the carbon chain may be branched or straight, with straight being preferred. Specifically, the alkoxy groups comprise C1-C6 alkoxy, more specifically C1-C4 alkoxy most specifically methoxy.

(E)-α,β unsaturated aryl sulfones may be prepared by Knoevenagel condensation of aromatic aldehydes with benzylsulfonyl acetic acids or arylsulfonyl acetic acids. The procedure is described by Reddy et al., Acta. Chim. Hung. 115:269-71 (1984); Reddy et al., Sulfur Letters 13:83-90 (1991); Reddy et al., Synthesis No. 4, 322-23 (1984); and Reddy et al., Sulfur Letters 7:43-48 (1987). See, particularly, the entire disclosures of U.S. Patent Nos. 6,656,973 and 6,667,346.

The α,β unsaturated aryl sulfones may take the form or pharmaceutically acceptable salts. The term "pharmaceutically acceptable salts" embraces salts commonly used to form alkali metal salts and to form addition salts of free acids or free bases. The nature of the salt is not critical, provided that it is pharmaceutically-acceptable.

**Table 1: Example α,β unsaturated aryl sulfone Compounds**

| **Example** | **Compound** |
|---|---|
| 1 | (E)-styryl phenyl sulfone |
| 2 | (E)-4-chlorostyryl phenyl sulfone |
| 3 | (E)-2,4-dichlorostyryl phenyl sulfone |
| 4 | (E)-4-bromostyryl phenyl sulfone |
| 5 | (E)-4-chlorostyryl 4-chlorophenyl sulfone |
| 6 | (E)-4-methylstyryl 4-chlorophenyl sulfone |
| 7 | (E)-4-methoxystyryl 4-chlorophenyl sulfone |
| 8 | (E)-4-bromostyryl 4-chlorophenyl sulfone |
| 9 | (E)-2-chlorostyryl benzyl sulfone |
| 10 | (E)-4-chlorostyryl benzyl sulfone |
| 11 | (E)-4-fluorostyryl 4-chlorobenzyl sulfone |
| 12 | (E)-4-chlorostyryl 4-chlorobenzyl sulfone |
| 13 | (E)-4-fluorostyryl 4-fluorobenzyl sulfone |
| 14 | (E)-2,4-difluorostyryl 4-fluorobenzyl sulfone |
| 15 | (E)-4-fluorostyryl 4-bromobenzyl sulfone |
| 16 | (E)-4-bromostyryl 4-bromobenzyl sulfone |
| 17 | (E)-bromostyryl 4-fluorobenzyl sulfone |
| 18 | (E)-4-chlorostyryl 4-bromobenzyl sulfone |
| 19 | (E)-4-bromostyryl 4-chlorobenzyl sulfone |
| 20 | (E)-4-fluorostyryl-4-trifluoromethylbenzylsulfone |
| 21 | (E)-4-chlorostyryl-4-trifluoromethylbenzylsulfone |
| 22 | (E)-4-bromostyryl-4-trifluoromethylbenzylsulfone |
| 23 | (E)-4-fluorostyryl-2,4-dichlorobenzylsulfone |
| 24 | (E)-4-chlorostyryl-2,4-dichlorobenzylsulfone |
| 25 | (E)-4-fluorostyryl-3,4-dichlorobenzylsulfone |
| 26 | (E)-4-chlorostyryl-3,4-dichlorobenzylsulfone |
| 27 | (E)-4-bromostyryl-3,4-dichlorobenzylsulfone |
| 28 | (E)-4-bromostyryl-4-nitrobenzylsulfone |
| 29 | (E)-4-fluorostyryl-4-cyanobenzylsulfone |
| 30 | (E)-4-chlorostyryl-4-cyanobenzylsulfone |
| 31 | (E)-4-bromostyryl-4-cyanobenzylsulfone |
| 32 | (E)-3,4-difluorostyryl-4-chlorobenzylsulfone |
| 33 | (E)-3-chloro-4-fluorostyryl-4-chlorobenzylsulfone |
| 34 | (E)-2-chloro-4-fluorostyryl-4-chlorobenzylsulfone |
| 35 | (E)-2,4-dichlorostyryl-4-chlorobenzylsulfone |
| 36 | (E)-3,4-dichlorostyryl-4-chlorobenzylsulfone |
| 37 | (E)-2,3-dichlorostyryl-4-chlorobenzylsulfone |
| 38 | (Z)-styryl benzylsulfone |
| 39 | (Z)-styryl 4-chlorobenzylsulfone |
| 40 | (Z)-styryl 2-chlorobenzylsulfone |
| 41 | (Z)-styryl 4-fluorobenzylsulfone |
| 42 | (Z)-4-chlorostyryl benzylsulfone |
| 43 | (Z)-4-chlorostyryl 4-chlorobenzylsulfone |
| 44 | (Z)-4-chlorostyryl 2-chlorobenzylsulfide |
| 45 | (Z)-4-chlorostyryl 4-fluorobenzylsulfone |
| 46 | (Z)-4-fluorostyryl benzylsulfone |
| 47 | (Z)-4-fluorostyryl4-chlorobenzylsulfone |
| 48 | (Z)-4-fluorostyryl 2-chlorobenzylsulfone |
| 49 | (Z)-4-fluorostyryl4-fluorobenzylsulfone |
| 50 | (Z)-4-bromostyryl benzylsulfone |
| 51 | (Z)-4-bromostyryl 4-chlorobenzylsulfone |
| 52 | (Z)-4-bromostyryl 2-chlorobenzylsulfone |
| 53 | (Z)-4-bromostyryl 4-fluorobenzylsulfone |
| 54 | (Z)-4-methylstyryl benzylsulfone |
| 55 | (Z)-4-methylstyryl 4-chlorobenzylsulfone |
| 56 | (Z)-4-methylstyryl 2-chlorobenzylsulfone |
| 57 | (Z)-4-methylstyryl 4-fluorobenzylsulfone |
| 58 | (E)-2-nitrostyryl-4-fluorobenzylsulfone |
| 59 | (E)-3-nitrostyryl-4-fluorobenzylsulfone |
| 60 | (E)-4-nitrostyryl-4-fluorobenzylsulfone |
| 61 | (E)-2-trifluoromethylstyryl-4-fluorobenzylsulfone |
| 62 | (E)-3-trifluoromethylstyryl-4-fluorobenzylsulfone |
| 63 | (E)-4-trifluoromethylstyryl-4-fluorobenzylsulfone |
| 64 | (E)-2-trifluoromethyl-4-fluorostyryl-4-fluorobenzylsulfone |
| 65 | (E)-2-nitrostyryl-4-chlorobenrylsulfone |
| 66 | (E)-3-nitrostyryl-4-chlorobenzylsulfone |
| 67 | (E)-4-nitrostyryl-4-chlorobenzylsulfone |
| 68 | (E)-2-trifluoromethylstyryl-4-chlorobenzylsulfone |
| 69 | (E)-3-trifluoromethylstyryl-4-chlorobenzylsulfone |
| 70 | (E)-4-trifluoromethylstyryl-4-chlorobenzylsulfone |
| 71 | (E)-2-trifluoromethyl-4-fluorostyryl-4-chlorobenzylsulfone |
| 72 | (E)-3-methyl-4-fluorostyryl-4-chlorobenrylsulfone |
| 73 | (E)-2-nitrostyryl-2,4-dichlorobenzylsulfone |
| 74 | (E)-2-trifluoromethyl-4-fluorostyryl-2,4-dichlorobenzylsulfone |
| 75 | (E)-2-nitrostyryl-4-bromobenzylsulfone |
| 76 | (E)-3-nitrostyryl-4-bromobenzylsulfone |
| 77 | (E)-4-nitrostyryl-4-bromobenzylsulfone |
| 78 | (E)-2-trifluoromethylstyryl-4-bromobenzylsulfone |
| 79 | (E)-3-trifluoromethylstyryl-4-fluorobenzylsulfone |
| 80 | (E)-4-trifluoromethylstyryl-4-bromobenzylsulfone |
| 81 | (E)-2-nitrostyryl-4-cyanobenzylsulfone |
| 82 | (E)-3-nitrostyryl-4-cyanobenzylsulfone |
| 83 | (E)4-nitrostyry]4-cyanobenzylsulfone |
| 84 | (E)-4-fluorostyryl-4-methylbenzylsulfone |
| 85 | (E)-4-bromostyryl-4-methylbenzylsulfone |
| 86 | (E)-2-nitrostyryl-4-methylbenzylsulfone |
| 87 | (E)-3-nitrostyryl-4-methylbenzylsulfone |
| 88 | (E)-4-nitrostyryl-4-methylbenzylsulfone |
| 89 | (E)-4-fluorostyryl- 4-methoxybenzylsulfone |
| 90 | (E)-4-chlorostyryl-4-methoxybenzylsulfone |
| 91 | (E)-4-bromostyryl-4-methoxybenzylsulfone |
| 92 | (E)-2-nitrostyryl-4-methoxybenzylsulfone |
| 93 | (E)-3-nitrostyryl-4-methoxybenzylsulfone |
| 94 | (E)-4-nitrostyryl-4-methoxybenzylsulfone |
| 95 | (E)-4-chlorostyryl-4-nitrobenzylsulfone |
| 96 | (E)-4-fluorostyryl-4-nitrobenzylsulfone |
| 97 | (E)-2,3,4,5,6-pentafluorostyryl-4-fluorobenzylsulfone |
| 98 | (E)-2,3,4,5,6-pentafluorostyryl-4-chlorobenzylsulfone |
| 99 | (E)-2,3,4,5,6-pentafluorostyryl-4-bromobenzylsulfone |
| 100 | (E)-4-fluorostyryl-2,3,4,5,6-pentafluorobenzylsulfone |
| 101 | (E)-4-chlorostyryl-2,3,4,5,6-pentafluorobenzylsulfone |
| 102 | (E)-4-bromostyryl-2,3,4,5,6-pentafluorobenzylsulfone |
| 103 | (E)-2,3,4,5,6-pentafluorostyryl-3,4-dichlorobenzylsulfone |
| 104 | (E)-2,3,4,5,6-pentafluorostyryl-2,3,4,5,6-pentafluorobenzylsulfone |
| 105 | (E)-2,3,4,5,6-pentafluorostyryl-4-iodobenrylsulfone |
| 106 | (E)-2-hydroxy-3,5-dinitrostyryl-4-fluorobenzylsulfone |
| 107 | (E)-2-hydroxy-3,5-dinitrostyryl-4-bromobenzylsulfone |
| 108 | (E)-2-hydroxy-3,5-dinitrostyryl-4-chlorobenzylsulfone |
| 109 | (E)-2-hydroxy-3,5-dinitrostyryl-2,4-dichlorobenzylsulfone |
| 110 | (E)-2,4,6-trimethoxystyryl-4-methoxybenzylsulfone |
| 111 | (E)-3-methyl-2,4-dimethoxystyryl-4-methoxybenzylsulfone |
| 112 | (E)-3,4,5-trimethoxystyryl-4-methoxybenzylsulfone |
| 113 | (E)-3,4,5-trimethoxystyryl-2-nitro-4,5-dimethoxybenzylsulfone |
| 114 | (E)-2,4,6-trimethoxystyryl-2-nitro-4,5-dimethoxybenzylsulfone |
| 115 | (E)-3-methyl-2,4-dimethoxystyryl-2-nitro-4,5-dimethoxybenrylsulfone |
| 116 | (E)-2,3,4-trifluorostyryl-4-fluorobenzylsulfone |
| 117 | (E)-2,3,4-trifluorostyryl-4-chlorobenzylsulfone |
| 118 | (E)-2,6-dimethoxy-4-hydroxystyryl-4-methoxybenzylsulfone |
| 119 | (E)-2,3,5,6-tetrafluorostyryl-4-methoxybenzylsulfone |
| 120 | (E)-2,4,5-trimethoxystyryl-4-methoxybenzylsulfone |
| 121 | (E)-2,3,4-trimethoxystyryl-4-methoxybenzylsulfone |
| 122 | (E)-3-nitro-4-hydroxy-5-methoxystyryl-4-methoxybenzylsulfone |
| 123 | (E)-3,4-dimethoxy-6-nitrostyryl-4-methoxybenzylsulfone |
| 124 | (E)-3,4-dimethoxy-5-iodostyryl-4-methoxybenzylsulfone |
| 125 | (E)-2,6-dimethoxy-4-fluorostyryl-4-methoxybenzylsulfone |
| 126 | (E)-2-hydroxy-4,6-dimethoxystyryl-4-methoxybenzylsulfone |
| 127 | (E)-2,4,6-trimethylstyryl-4-methoxybenzytsulfone |
| 128 | (E)-2,4,6-trimethoxystyryl-4-chlorobenzylsul fone |
| 129 | (E)-2,6-dimethoxy-4-fluorostyryl-4-chlorobenzylsulfone |
| 130 | (E)-2-hydroxy-4,6-dimethoxystyryl-4-chlorobenzylsulfone |
| 131 | (E)-2,4,6-trimethoxystyryl-4-bromobenzylsulfone |
| 132 | (E)-2,6-dimethoxy-4-fluorostyryl-4-bromobenzylsulfone |
| 133 | (E)-2,4,6-trimethoxystyryl-2,3,4-trimethoxybenzylsulfone |
| 134 | (E)-2,6-dimethoxystyryl-2,3,4-trimethoxybenzylsulfone |
| 135 | (E)-2,4,6-trimethoxystyryl-,3,4,5-trimethoxybenzylsulfone |
| 136 | (E)-2,6-dimethoxystyryl-3,4,5-trimethoxybenzylsulfone |
| 137 | (E)-4-fluorostyryl-2,3,4-trimethoxybenzylsulfone |

In one exemplary alternative, α,β unsaturated aryl sulfone formula 1a: 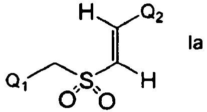 wherein Q₁ and Q₂ are, same or different, are substituted or unsubstituted aryl, or substituted or unsubstituted heteroaryl illustrated by Table 2 below.

**Table 2: Formula 1a Wherein Q₁ And Q₂ Are:**

| **Example** | **Q₁** | **Q₂** |
|---|---|---|
| 138 | 4-fluorophenyl | 2-pyridyl |
| 139 | 4-fluorophenyl | 3-pyridyl |
| 140 | 4-fluorophenyl | 4-pyridyl |
| 141 | 4-chlorophenyl | 2-pyridyl |
| 142 | 4-chlorophenyl | 3-pyridyl |
| 143 | 4-chlorophenyl | 4-pyridyl |
| 144 | 4-bromophenyl | 2-pyridyl |
| 145 | 4-bromophenyl | 3-pyridyl |
| 146 | 4-bromophenyl | 4-pyridyl |
| 147 | 4-fluorophenyl | 2-thienyl |
| 148 | 4-chlorophenyl | 2-thienyl |
| 149 | 4-bromophenyl | 2-thienyl |
| 150 | 4-fluorophenyl | 4-bromo-2-thienyl |
| 151 | 4-chlorophenyl | 4-bromo-2-thienyl |
| 152 | 4-bromophenyl | 4-bromo-2-thienyl |
| 153 | 4-fluorophenyl | 5-bromo-2-thienyl |
| 154. | 4-chlorophenyl | 5-bromo-2-thienyl |
| 155 | 4-bromophenyl | 5-bromo-2-thienyl |
| 156 | 4-fluorophenyl | 2-thienyl-1,1-dioxide |
| 157 | 4-chlorophenyl | 2-thienyl-1,1-dioxide |
| 158 | 4-bromophenyl | 2-thienyl-1,1-dioxide |
| 159 | 4-fluorophenyl | 3-thienyl |
| 160 | 4-chlorophenyl | 3-thienyl |
| 161 | 4-bromophenyl | 3-thienyl |
| 162 | 4-iodophenyl | 3-thienyl |
| 163 | 4-methylphenyl | 3-thienyl |
| 164 | 4-methoxyphenyl | 3-thienyl |
| 165 | 4-trifluoro-methylphenyl | 3-thienyl |
| 166 | 2,4-dichlorophenyl | 3-thienyl |
| 167 | 3,4-dichlorophenyl | 3-thienyl |
| 168 | 4-cyanophenyl | 3-thienyl |
| 169 | 4-nitrophenyl | 3-thienyl |
| 170 | 4-fluorophenyl | 3-thienyl-1,1-dioxide |
| 171 | 4-chlorophenyl | 3-thienyl-1,1-dioxide |
| 172 | 4-bromophenyl | 3-thienyl-1,1-dioxide |
| 173 | 4-methoxyphenyl | 3-thienyl-1,1-dioxide |
| 174 | 2,4-dichlorophenyl | 3-thienyl-1,1-dioxide |
| 175 | 4-fluorophenyl | 2-furyl |
| 176 | 4-chlorophenyl | 2-furyl |
| 177 | 4-bromophenyl | 2-furyl |
| 178 | 4-fluorophenyl | 3-furyl |
| 179 | 4-chlorophenyl | 3-furyl |
| 180 | 4-bromophenyl | 3-furyl |
| 181 | 4-iodophenyl | 3-furyl |
| 182 | 4-methylphenyl | 3-furyl |
| 183 | 4-methoxyphenyl | 3-furyl |
| 184 | 4-trifluoro-methylphenyl | 3-furyl |
| 185 | 2,4-dichlorophenyl | 3-furyl |
| 186 | 3,4-dichlorophenyl | 3-furyl |
| 187 | 4-cyanophenyl | 3-furyl |
| 188 | 4-nitrophenyl | 3-furyl |
| 189 | 4-chlorophenyl | 2-thiazolyl |
| 190 | 4-chlorophenyl | 2-pyrrolyl |
| 191 | 4-bromophenyl | 2-pyrrolyl |
| 192 | 4-chlorophenyl | 2-nitro-4-thienyl |
| 193 | 4-iodophenyl | 2-nitro-4-thienyl |
| 194 | 2,4-dichlorophenyl | 2-nitro-4-thienyl |
| 195 | 4-methoxyphenyl | 2-nitro-4-thienyl |
| 196 | 4-fluorophenyl | 1-naphthyl |
| 197 | 4-fluorophenyl | 2-naphthyl |
| 198 | 4-chlorophenyl | 1-naphthyl |
| 199 | 4-chlorophenyl | 2-naphthyl |
| 200 | 4-bromophenyl | 1-naphthyl |
| 201 | 4-bromophenyl | 2-naphthyl |
| 202 | 1-naphthyl | 4-fluorophenyl |
| 203 | 1-naphthyl | 4-chlorophenyl |
| 204 | 1-naphthyl | 4-bromophenyl |
| 205 | 1-naphthyl | 2-nitrophenyl |
| 206 | 1-naphthyl | 3-nitrophenyl |
| 207 | 1-naphthyl | 4-nitrophenyl |
| 208 | 4-fluorophenyl | 9-anthryl |
| 209 | 4-chlorophenyl | 9-anthryl |
| 210 | 4-bromophenyl | 9-anthryl |

**Table 3**

| **Example** | **Compound** |
|---|---|
| 211 | (E)-styryl phenyl sulfone |
| 212 | (E)-4-chlorostyryl phenyl sulfone |
| 213 | (E)-2,4-dichlorostyryl phenyl sulfone |
| 214 | (E)-4-bromostyryl phenyl sulfone |
| 215 | (E)-4-chlorostyryl 4-chlorophenyl sulfone |
| 216 | (E)-4-methylstyryl 4-chlorophenyl sulfone |
| 217 | (E)-4-methoxystyryl 4-chlorophenyl sulfone |
| 218 | (E)-4-bromostyryl 4-chlorophenyl sulfone |
| 219 | (E)-2-chlorostyryl benzyl sulfone |

Polymorphs of all compounds disclosed and contemplated herein are intended to be within the disclosure.

### II. EXAMPLE SPECIES

The radioprotective α,β unsaturated arylsulfone contained in the formulation of the invention is **ON.1210.Na.** ON.1210.Na is a derivative of chlorobenzylsulfone. This and related compounds are described herein as exhibiting valuable prophylactic properties which mitigate the effects of accidental and intentional exposure to life-threatening levels of irradiation. Hence, a systematic development of this and related compounds is described with the objective of developing a shelf stable formulations.

Table 4 describes the general physical properties of ON.1210.Na. The compound is a sodium salt of (E)-4-Carboxystyryl-4-chlorobenzylsulfone.

**Table 4: Physical Properties of ON.1210.Na**

| | |
|---|---|
| Chemical Structure | 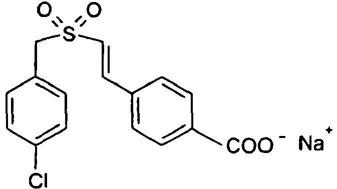 |
| Chemical Name | (E)-4-Carboxystyryl-4-chlorobenzylsulfone, Sodium Salt |
| Empirical Formula | C₁₆H₁₂ClNaO₄S |
| Molecular Weight | 358.79 |
| Physical Nature | White crystalline flakes |
| Melting Point | 354-356°C |
| Solubility | Soluble in water at 8-10 mg/ml |

The compound ON 01210.Na appears to form at least one polymorph. X-ray diffraction pattern, for example, of precipitated ON 01210.Na is different from that of the originally synthesized compound. Polymorphs of ON 01210.Na are intended to be within the scope of the claims appended hereto.

Treatment of normal human fibroblasts with ON 01210.Na, for example, prior to exposure to cytotoxic levels of ionizing radiation provides dose-dependent radio-protection.

The physio-chemical properties of ON.1210.Na, as an example drug substance, was determined in order to evaluate appropriate formulation approaches for development of a safe, reliable and effective parenteral formulation of these compounds. This includes microscopic studies, partition coefficient, pKa, pH solubility studies, pH stability studies under accelerated conditions, solid state characterization of the drug substance, and solid state stability of drug substance under accelerated conditions. *See,* section IV, *infra.* This example compound has a low octanol:water partition coefficient (1.28-2.87). The equilibrium solubility of the drug at pH 4.0, 5.0, 6.0, 7.4, 8.0, 9.0 was 0.000154, 0.0379, 0.715, 11.09, 16.81, 23.3 mg/mL, respectively. The pKa calculated from pH-solubility studies was 2.85 ± 0.6. The pH-stability profile of the drug indicated better stability at neutral and biological pH but degradation was rapid under acidic conditions. The degradation followed a pseudo-first-order rate. The accelerated solid-state stability study of the bulk drug substance showed no evidence of degradation. This drug is quite stable in an aqueous environment at biological pH. Therefore, it can be formulated as a shelf stable parenteral formulation. The aqueous solubility of the drug as the free acid is low and can be significantly enhanced by increase in pH, co-solvents and complexation.

### III. FORMULATIONS OF RADIOPROTECTANT α,β UNSATURATED ARYL SULFONES

Although compositions described and contempated herein are not so limited, due to the oral, for example, bioavailability of the compounds upon administration, a preferred route of administration of the compositions described herein include, for example, parenteral administration. Parenteral administration includes intravenous, intramuscular, intraarterial, intraperitoneal, intravaginal, intravesical (e.g., into the bladder), intradermal, topical or subcutaneous administration. The α,β unsaturated aryl sulfone may be administered in the form of a pharmaceutical composition comprising one or more α,β unsaturated aryl sulfones in combination with a pharmaceutically acceptable carrier. By "pharmaceutically acceptable carrier" is meant any carrier, diluent or excipient which is compatible with the other ingredients of the formulation and is not deleterious to the subject.

The specific dose and schedule of α,β unsaturated aryl sulfone to obtain the radioprotective benefit will, of course, be determined by the particular circumstances of the individual patient including, the size, weight, age and sex of the patient, the nature and stage of the disease being treated, the aggressiveness of the disease, and the route of administration, and the specific toxicity of the radiation. For example, a daily dosage of from about 0.01 to about 150 mg/kg/day may be utilized, more preferably from about 0.05 to about 50 mg/kg/day. Particularly preferred are doses from about 1.0 to about 10.0 mg/kg/day, for example, a dose of about 7.0 mg/kg/day. The dose may be given over multiple administrations, for example, two administrations of 3.5 mg/kg. Higher or lower doses are also contemplated.

Shelf-stable pharmaceutical compositions which comprise effective amount of at least one radioprotective α, β unsaturated aryl sulfone compound are described herein. The term "effective amount" of at least one radioprotective α, β unsaturated aryl sulfone compound, for example, as used herein refers to an amount of compound, after dilution as described *infra,* that is effective to mitigate, reduce or eliminate toxicity associated with radiation in normal cells of the subject and/or to impart a direct cytotoxic effect to abnormally proliferating cells in the subject. As used with respect to bone marrow purging, "effective amount of the radioprotective α,β unsaturated aryl sulfone compound" refers to an amount of at least one α,β unsaturated aryl sulfone effective to reduce or eliminate the toxicity associated with radiation in bone marrow removed from a subject and/or to impart a direct cytotoxic effect to malignant cells in the bone marrow removed from the subject.

The formulations described and claimed herein are generally intended for parenteral administration. Compositions of the present invention are generally formulated with active ingredient, in a concentrated form for storage and handling prior to dilution with suitable parenteral diluent prior to infusion.

A single dosage is generally within the range of about 1 ml to about 5 ml of any of the compositions described herein. Individual 3 ml dosages of compositions described herein are contemplated, for example. The dosages may be packaged, for example, in 5 ml vials. Compositions of the present invention may, for example, be diluted with about 7 parts diluent (7:1) prior to administration. However, the dilution factor and the diluent employed depend on the concentration of drug in the formulation. Compositions of the present invention, however, may be diluted with anywhere, for example, within the range of about 2 volumes of suitable parenteral diluent prior to infusion to about 12 volumes of suitable parenteral diluent, prior to administration.

Compositions of the present invention may be diluted, for example, for parenteral administration and should have a pH within the range of about 6.5 to about 10.0. Preferably the final diluted product for parenteral administration should have a pH within the range of about 7.0 to about 9.5 A *diluted* product pH of about 7.0 to about 8.0 is preferredThe osmolarity of the diluted formulation for parenteraladministration should be approximately within the range of about 200 to about 400 mOsm/kg. Preferred osmolarity of the diluted formulation for administration should be approximately within the range of about 270 to about 330 mOsm/kg. A preferred osmolarity of the diluted formulation for administration should be approximately 300 mOsm/kg.

Compositions described herein, unless specified otherwise, are aqueous-based.

### SOLUTIONS

The compositions of the present invention comprise between 20 mg/ml to 100 mg/ml (e.g., about 25mg/ml, 50mg/ml, 75 mg/mi, 100 mg/ml, ) of at least one α, β unsaturated aryl sulfone ((E)-4-carboxystyryl-4-chlorobenzylsulfone sodium salt (ON.1210.Na)); wherein the composition exhibits a pH within the range of 8.3 to 8.7. About 8.5 is an example preferred pH (between 8.4 and 8.6). The buffer is Tris-EDTA which provides a good physiological buffering capacity at the pH of administration. Exemplary compositions of the present invention described herein exhibit a final concentration of about 0.1M Tris and 0.01MEDTA. However, the Tris concentration may be within a range of 0.1 M to 0.3 M to suit the conditions for administration.

An example solution composition of the present invention comprises an effective amount of at least one radioprotective α, β unsaturated aryl sulfone compound in a formulation which comprises between 20 mg/ml to 100mg/ml of the compound (ON.1210.Na); at least one buffer of Tris-EDTA; PEG 400 within the range of 40% w/v to 60% w/v (e.g., 50% w/v); and, wherein the composition has a pH within the range of 8.3 to 8.7 (e.g., between 8.4 and 8.6).

An example composition of the present invention comprises 20mg/ml⁻ to 60mg/ml of the compound (ON.1210.Na); Tris-EDTA buffer within the range of 0.15M to 0.25M; PEG 400 within the range of 45% w/v to 55% w/v; and, wherein the composition has a pH within the range of 8.4 to 8.6, and the compound is substantially stable in the composition at about 25°C for at least about 120 days.

High pH, e.g., about 8.5, is preferred. The compositions comprise between 40% and 60% of PEG 400, e.g. at least 40%, at least 45%, at least 50%, at least 55%.

A solution formulation of the present invention comprises Tris-EDTA buffer composed of Tris within the range of 0.1M to 0.3M and EDTA within the range of about 0.01M to about 0.03M ; PEG within the range of 40% w/v to 60% w/v; and, wherein the composition has a pH within the range of 8.3 to 8.7. An exemplary composition comprises between 20mg/ml to 60mg/ml of the compound (ON.1210.Na); Tris-EDTA composed of Tris within the range of 0.15M to 0.25M and EDTA within the range of 0.015M to 0.025M; PEG 400 within the range of 45% w/v to 55% w/v; and, wherein the composition has a pH within the range of 8.4 to 8.6, and the compound is substantially stable in the composition at about 25°C for at least about 120 days.

Further identified and disclosed herein is the fact that the half life of α, β unsaturated aryl sulfones, ON.1210.Na, for example, increases significantly when the route of administration is changed to intramuscular (in contrast to intravenous), and increases even further upon subcutaneous administration.

### ALTERNATE ELEMENTS FOR CURRENT FORMULATIONS

The term "water soluble polymer", as used herein, for use in alternate formulations of the present invention includes but is not limited to water soluble polymer excipients known in the art including poly-oxyethylene, poly-oxyethylene-poly-oxypropylene copolymer, polyvinylalcohol, polyvinylpyrrolidone (PVP) (Povidone (soluble homopolymer of N-vinyl-2-pyrrolidone)), polyvinylpyridine N-oxide, copolymer of vinylpyridine N-oxide and vinylpyridine, as well as derivatives thereof, and combinations thereof. Poly-oxyethylene and/or poly-oxyethylene-poly-oxypropylene copolymers are example water-soluble polymers for use in alternate formulations of the present invention. Poloxamer 407 (e.g., Pluronic F 127, Lutrol® micro 127), for example, and/or Poloxamer 188 (e.g., Pluronic F 68, Lutrol® micro 68) are poly-oxyethylene-poly-oxypropylene copolymers that can be used independently or in combination in formulations of the present invention. BASF Corporation, Mount Olive, NJ.

Polyethylene glycols (PEGs) are preferred water soluble polymers. Low molecular weight liquid polyethylene glycols, for example, PEG 300, PEG 400, PEG 600, and PEG 800, are preferred water soluble polymers that can be used independently or in combination with each other, for example, in formulations of the present invention. Particularly preferred are PEG 300, PEG 400, and PEG 600. Lutrol® E 300, Lutrol® E 400 and Lutrol® E 600, for example, are commercially available from BASF Corporation, Mount Olive, NJ. PEG 400 (Polyethylene glycol 400, Macrogol 400, PEG 400, Poly(oxy-1,2-ethanediyl),alpha-hydro-omega-hydroxy- (CAS No: 25322-68-3)), e.g., Lutrol® E 400, is preferred. Example water soluble polymers selected from the group consisting essentially of PEG 300, PEG 400, PEG 600, and PEG 800 are preferred. Although not specifically listed here PEG products substantially the same, otherwise within this characteristic range of PEG entities, may be employed in compositions of the present invention.

Low molecular weight povidone grades (CAS No: 9003-39-8), Kollidon® 12 PF and Kollidon® 17 PF, for example, form a soluble complex between the compounds and povidone. Both products are available as pyrogen-free powders, suitable for use in injectables, from BASF Corporation, Mount Olive, NJ. Kollidon® 12 PF, Kollidon® 17 PF, Kollidon® 25, Kollidon® 30, and Kollidon® 90 F are preferred examples of povidone. Compositions of the present invention are exemplified herein that comprise Povidone K 90, for example, at concentrations of about 1% to about 5% in water. These compositions are further contemplated, for example, that comprise Povidone K 90, for example, at concentrations of about 0.5% to about 10% in water. Low molecular weight Povidones, for example, may be employed at much higher concentrations in compositions of the present invention, e.g., between about 5% by weight and about 40%. A preferred range is between about 5% to about 20% by weight, depending on the viscosity of the formulation.

Chemically Modified Cyclodextrins are example components of compositions described herein. Cyclodextrin drug compositions described herein are easily diluted, for example, eliminating the need for elaborate mixing procedures. Chemically modified cyclodextrin, hydroxypropyl-b-cyclodextrin, for example, formulations are preferred for parenteral administration due to the ability to of the cyclodextrins to solubilize and stabilize the compounds described herein as well as the superior safety profile exhibited by the chemically modified cyclodextrins. The use of cyclodextrins referred to herein can reduce dosing volume and *in situ* irritation resulting from high pH, for example, other solvents, or any direct chemical irritancy due to the compounds otherwise described herein.

Many different chemical moieties may be introduced into the Cyclodextrin molecule by reaction with the hydroxyl groups lining the upper and lower ridges of the toroid; for example, hydroxypropyl, carboxymethyl, and acetyl. Since each Cyclodextrin hydroxyl groups differs in its chemical reactivity, reaction processes produces an amorphous mixture of thousands of positional and optical isomers. Preferred examples of chemically modified cyclodextrins as components of formulations of the present invention include, but are not limited to, 2-hydroxypropyl-beta-Cyclodextrin, 2-hydroxypropyl-gamma-Cyclodextrin, and hydroxyethyl-beta-Cyclodextrin. Cyclodextrin molecules (alpha, beta, or gamma) can have up to 3(n) substituents, where n is the number of glucopyranose units of the Cyclodextrin molecule. This is referred to as the degree of substitution (DS). The DS refers to substituents other than hydrogen; substituents may be all of one kind or mixed. Non-integer degrees of substitution occur as weighted averages are used to describe substitutional variability. See, e.g., Volume 3 (cyclodextrins) of the 11 Volume Collection "Comprehensive Supramolecular Chemistry", available through Elsevier Science Inc., 660 White Plains road, Tarrytown, N.Y., 10591-5153 USA. *See, also,* Pitha, Josef, U.S. Patent No. 4,727,064; *Pharmaceutical Preparations Containing Cyclodextrin Derivatives;* Muller, B.W., U.S. Patent No. 4,764,604, *Derivatives of Gamma Cyclodextrins;* Yoshida, A., et al., (1988) Int. Pharm, Vol. 46, p. 217: Pharmaceutical Evaluation Of Hydroxy Alkyl Ethers Of B-Cyclodextrins*;* Muller, B. W., (1986). J. Pharm Sci. 75, No 6, June 1986: Hydroxypropyl-B-Cyclodextrin Derivatives: Influence Of Average Degree Of Substitution On Complexing Ability And Surface Activity*;* Irie, T., et al., (1988) Pharm Res., No 11, p. 713: Amorphous Water- Soluble Cyclodextrin Derivatives: 2-hydroxyethyl, 3-hydroxypropyl, 2-hyroxyisobutyl, and carboxamidomethyl derivatives of B-cyclodextrin*.*

Hydroxypropyl-B-Cyclodextrin (hydroxypropyl cyclodextrin) (HPCD) is itself very soluble in water (greater than 500 mg/ml at room temperature). Dr. Joseph Pitha of the NIH has experimentally evaluated many of the uses of this cyclodextrin derivative and found that it can be conveniently applied to cell cultures and membrane preparations. It is also observed that HPCD is non-toxic after IP and IV administration to different rodent species. The maximum human dose of HPCD given parenterally was approximately 500 mg/kg iv given continuously as a 5 percent aqueous solution to one individual for four days; no adverse clinical effects were reported. Pitha, Josef, et al., (1988) Life Sciences. 43, No 6, 493-502: *Drug Solubilizers To Aid Pharmacologists: Amorphous Cyclodextrin Derivatives.*

Compounds described herein may be formulated in compositions which generally comprise an effective amount of at least one α, β unsaturated aryl sulfone, water, and at least one chemically modified cyclodextrin. Particularly preferred compositions for the present invention comprise about 20% to about 60% w/v chemically modified cyclodextrin(s). Example compositions for the present invention comprise about 30% to about 50% w/v chemically modified cyclodextrin(s). An example composition of the present invention comprises at least one chemically modified cyclodextrin selected from the group consisting of 2-hydroxypropyl-beta-Cyclodextrin, 2-hydroxypropyl-gamma-Cyclodextrin, and hydroxyethyl-beta-Cyclodextrin, preferably in an amount of about 30% to about 50% w/v, an effective amount of at least one α, β unsaturated aryl sulfone, (E)-4-carboxystyryl-4-chlorobenzylsulfone sodium salt (ON.1210.Na), and water. 2-hydroxypropyl-beta-Cyclodextrin, (Hydroxypropyl-B-Cyclodextrin) (hydroxypropyl cyclodextrin) (HPCD) is a preferred chemically modified cyclodextrin for use in compositions of the present invention. Shelf-stable aqueous compositions for the present invention, for dilution prior to parenteral administration for the protection of normal cells from toxicity of ionizing radiation or to mitigate the effects of accidental/intentional exposure to life-threatening levels of irradiation, comprise, for example, an effective amount of at least one α, β unsaturated aryl sulfone, and about 35% to about 45% (e.g., about 40% w/v) w/v chemically modified cyclodextrin.

Preferred shelf-stable compositions of the present invention, for dilution prior to administration, comprise between about 20 mg/ml to about 100 mg/ml of the α, β unsaturated aryl sulfone. Particularly preferred compositions of the present invention comprise between about 20 mg/ml to about 80 mg/ml of the α, β unsaturated aryl sulfone. Example compositions of the present invention comprise between about 20 mg/ml to about 75 mg/ml (e.g., about 25mg/ml, 50mg/ml, 75mg/ml) of the α, β unsaturated aryl sulfone ((E)-4-carboxystyryl-4-chlorobenzylsulfone sodium salt (ON.1210.Na)); and, wherein the composition has a pH within the range of 8.3 to 8.7 (e.g., about 8.5).

Preferred formulations described and claimed herein have significantly increased the solubility of radioprotective α, β unsaturated aryl sulfones, ON.1210.Na, for example, to allow concentrated formulations of the present invention, wherein upon dilution, the drug is physically stable for at least about 24 hours.

### IV. FORMULATION STUDIES

ON.1210.Na, a sodium salt of a chlorobenzylsulphone, is an example of an efficacious radiation protectant drug. ON.1210.Na is particularly shown to protect during life-threatening levels of radiation exposure. Described herein are shelf stable parenteral formulations of this and related compounds for therapeutic administration to patients.

A stability indicating HPLC assay is used during the preformulation studies. Preformulation studies include determination of microscopic and macroscopic properties, partition coefficient, pKa, pH-solubility, pH-stability, solid-state characterization and solid-state stability. XRD and thermal analyses re used to characterize the solid and solid-state stability. The solid-state stability as well as the pH stability of the drug is carried out at 75°C.

Microscopic and XRD data reveals that the drug is crystalline having an irregular plate like crystals. The drug has a low octanol:water partition coefficient (1.28-2.87). The equilibrium solubility of the drug at pH 4.0, 5.0, 6.0, 7.4, 8.0, 9.0 is 0.000154,0.0379, 0.715, 11.09, 16.81, 23.3 mg/mL, respectively. The pKa calculated from a pH-solubility study is 2.85 ± 0.6. The pH-stability profile of the drug indicats better stability at neutral and biological pH but degradation is rapid under acidic condition. The degradation followed a first-order rate. The accelerated solid-state stability of the bulk drug substance shows no evidence of degradation.

The solubility of the drug can be significantly increased with increase in pH. Also, the stability of the drug can be enhanced by buffering the aqueous solutions around pH 7. The drug is quite stable in an aqueous environment rendering itself for the development of a shelf stable formulation.

**Table 5: Saturated Solubility of ON.1210.Na in Different Solvent Systems**

| **Solvents used** | **Solubility (mg/ml)** | **pH of solution** | **Dilution** | **Remarks** |
|---|---|---|---|---|
| 20 % cyclodextran (Feb 4^{th}, 24 hours of equilibration) | 62 | 8.26 | | |
| 10 % cyclodextran | 46 | 8.14 | | |
| 5% cyclodextran | 39 | 8.32 | | |
| (Feb 5^{th}, 48 hours of equilibration) | | | | |
| 20 % cyclodextran | 58 | 8.35 | | |
| 10 % cyclodextran | 41 | 8.41 | | |
| 5% cyclodextran | 32 | 8.52 | | |
| Water | 11.8 | 8.40 | | |
| 10 % PEG400:water | 36 | 8.24 | 1:4 diluted with PBS (the filtered sol was diluted with PBS) | Solution turns hazy after 24 hrs |
| 25 % PEG400:water | 39 | 8.01 | | |
| 50 % PEG400:water | 40 | 7.53 | | |
| 50% PEG400:Buffer (0.07M) | 23.4 | 8.2 | | |
| 50% PEG400:Buffer (0.01M) | 20.8 | 8.2 | | |
| 100 % PEG 400 (12g/kg) | 26 | 8.34 | | |
| 100 % ethanol | 0.9 | 7.94 | | |
| 50 : 50 PEG400 : ethanol | 37 | 8.46 | | |
| 20 % HPCD:20% PEG 400:60% water | 53.6 | 8.2 | | |
| 20 % HPCD:20% PEG 400:60% water | 53.6 | 8.2 | | |
| PEG400:Benzyl alcohol (50:50) | 51.6 | 8.19 | | |
| Propylene Glycol | 44.0 | 7.27 | | |
| PEG300 (IP10g/kg) | 52.04 | 8.21 | | |
| 40% HPCD | 108.7 | 7.86 | | |
| For tween 80 in water | Solubility (mg/ml) | pH of solution | | |
| 0.25 % | 10.5 | 8.87 | | |
| 0.5 % | 10.3 | 8.74 | | |
| 1 % | 10.1 | 8.82 | | |
| 2 % | 9 | 8.78 | | |
| For Tween 80 in | Solubility | pH of | | |
| phosphate buffer | (mg/ml) | solution | | |
| 0.25 % | 9.5 | 8.45 | | |
| 0.5 % | 9.8 | 8.41 | | |
| 1 % | 8.9 | 8.45 | | |
| 2% | 9.7 | 8.39 | | |
| Propylene glycol: water (50:50) | 41.65 | 8.53 | | |
| PEG 300: water (50:50) | 30.6 | 4.5 | | |
| 1% (w/v) Povidone K 90 in water | 209.28 | 8.69 | | |
| 5% (w/v) Povidone K 90 in Water | 151.52 | 8.35 | | |
| NN-Dimethyl Acetamide:Water: PEG 400 (1:2:2) | 66.19 | 7.90 | | |
| 1% PVP C 30 | 25.1 | 8.02 | | Saturated solution on filtration kept at RT turns hazy after 24 hrs |
| 2% PVP C 15 | 26.9 | 8.69 | | |
| 1% PVP C 15 | 26.9 | 8.86 | | Saturated solution on filtration kept at RT turns hazy after 24 hrs |
| PEG300:ETH (70:30) | 48.3 | 8.99 | | Saturated solution on filtration kept at RT turns hazy immediately |
| PEG400:Ethanol 70:30 | 55.5 | 8.03 | | |
| DMA:WATER: Ethanol(1:1:1) | 46.4 | 8.11 | | |

**Table 6: Effect of Dilution on Solubility in different ON.1210.Na Formulations**

| **Formulation** | **Concentration (mg/ml)** | **pH of solution** | **Dilution** | **Remarks** |
|---|---|---|---|---|
| 50 mg/ml solution of ON.1210 in 40% HPCD | 44.99 | | 1. 1:4 (diluted with WFI) | No ppt. observed within 2 weeks |
| | | | 2. 1: 10 (diluted with WFI) | No ppt. observed within 2 weeks |
| | | | 3. 1:4 (diluted with PBS) | No ppt. observed within 2 weeks |
| 50 mg/ml solution of ON.1210 in DMA:Water:PEG400 (1:2:2) | 45.71 | | 1:4 (diluted with WFI) | Solution turns hazy after 24 hrs. |
| | | | 1:10 (diluted with WFI) | Solution turns hazy after 24 hrs. |
| | | | 1:4 (diluted with PBS | No immediate turbidity, Solution turns hazy after 24 hrs. |
| 30 mg/ml solution of ON.1210.Na in PEG300:Ethanol (70:30) | 27.89 | | 1:4 (diluted with WFI) | Solution turns hazy immediately. |
| | | | 1:10 (diluted with WFI) | Solution turns hazy immediately |
| | | | 1:4 (diluted with PBS) | No immediate turbidity, but turbidity obs within 24 hrs. |
| 30 mg/ml solution of ON.1210.Na in PEG400:Ethanol (70:30) | 27.48 | | 1:4 diluted with WFI | No immediate turbidity |
| | | | 1:10 diluted with WFI | No immediate turbidity |
| | | | 1:4 diluted with PBS | No immediate turbidity, also no turbidity after 24 hrs. |
| 50mg/ml ON.1210.Na solution was prepared in PEG 400:Water (70:30) | 39.55 | | 1:4 (diluted with WFI) | Solution turns hazy immediately. |
| | | | 1:10 (diluted with WFI) | Solution turns hazy immediately |
| | | | 1:4 (diluted with PBS) | No immediate turbidity, Solution turns hazy after 24 hrs. |
| 30mg/ml ON.1210.Na solution in DMA:WATER: Ethanol (1:1:1) | | | 1:4 (diluted with WFI) | Solution turns hazy immediately. |
| | | | 1:10 (diluted with WFI) | Solution turns hazy immediately |
| | | | 1:4 (diluted with PBS) | No immediate turbidity |

A study was conducted to develop and validate a stability indicating sensitive HPLC assay for ON.1210.Na for preformulation and formulation development. The isocratic system uses a mobile phase consisting of Acetonitrile:0.1% Trifluoroacetic acid in water (60:40 v:v) at a flow rate of 1 mL/min. A C-18 Gemini column (250 x 4.6 mm) is used and effluents are monitored at a 254 nm. Forced degradation is carried out by exposing ON.1210.Na to 0.1N HCl, 0.1N NaOH and 3% (v/v) hydrogen peroxide. The validation parameters include linearity, specificity, sensitivity, precision and accuracy.

Standard curves are linear in the concentration range of 0-500 µg/mL. The retention times of the drug and several degradation products were well within seven minutes. The Relative Standard Deviation (RSD) values for the within-day and day-to-day precision range from 0.4 to 2.5 % and 2.2 to 4.4 %, respectively. The RSD for accuracy measurement ranges from 0.85 to 1.7%. The critical level, the detection level and the determination level for this assay are 2.86 ± 0.67 µg/mL, 5.69 ± 0.67 µg/mL and 15.6 ± 1.79 µg/mL, respectively.

A sensitive and stability indicating HPLC method is developed and validated for ON.1210.Na. The force degradation, preformulation and formulation studies demonstrate the suitability of this method.

The first step in this process is the development of a stability indicating HPLC assay. The next phase is the validation of the assay. This HPLC assay is able to support preformulation, formulation development, and the stability studies of the bulk drug and formulated ON.1210.Na, for example.

### Stability of the ON.1210.Na (solid)

A solid drug sample (ON1210), after autoclaving in a crimped vial, is also evaluated for any degradation using the developed HPLC method. The sample, after autoclaving, is used to make a standard solution of 400 µg/mL and injected onto HPLC. The results indicate no reduction in the Area Under the Curve (AUC) of the parent peak, and also no additional peaks are detected. Based on this observation, it seems that the bulk drug ON.1210.Na is stable under the autoclaving conditions.

### Solid-State Stability of ON.1210.Na

The stability of ON.1210.Na in the solid state is determined by exposing the drug at 75°C over a prolonged time. The sample is crimped in a 10 mL injection vial and kept at the above temperature. The sample is collected after 15 days and one month and reconstituted with diluting solution and its concentration is determined by HPLC. Appearance of any additional peak in the chromatogram is also noted. A control sample is represented by ON.1210.Na not exposed to 75°C and reconstituted to a known concentration with diluting solution. The results of this study are depicted in Table 7.

**Table 7: Solid-State Stability of ON.1210.Na at 75°C**

| Day after exposure | Theoretical conc. (µg/mL) | Determined conc. (ug/mL) | % change | Sample not exposed to Heating condition | Theoretical conc. (ug/mL) | Determined concentration (ug/mL) | % change |
|---|---|---|---|---|---|---|---|
| 15 | 270 | 286.2 | +6 | (control) | 250 | 242.7 | -2.92 |
| 30 | 270 | 334.9 | +24 | (control) | 250 | 232.3 | -7.1 |
| 45 | 270 | 302.0 | +11.8 | (control) | 250 | 249.8 | -0.44 |
| 54 | 200 | 229.2 | +14.6 | (control) | 200 | 203.5 | +1.75 |

The solid state stability data shows that there is a slight increase in potency of the compound as a function of time at 75°C. This is probably due to the loss of moisture present in the sample. This was confirmed by determining the moisture content of the bulk ON.1210.Na. The result shows that there is about 14.5% of moisture in the sample which equates to 3 moles of water per mole of ON.1210.Na. The chromatograms showed no evidence of degradation of the sample.

### Thermal Analyses

A differential scanning calorimeter (DSC) (model DSC-50, Shimadzu, Kyoto, Japan) and a thermogravimetric analyzer (TGA) (model TGA-50, Shimadzu, Kyoto, Japan) are connected to a thermal analysis operating system (TA-50WS, Shimadzu, Kyoto, Japan). The heat of fusion is calibrated using indium (purity 99.99%; mp 156.4; ΔH 6.8 mcal/mg). The sample to be analyzed (5-10 mg) by DSC is crimped nonhermetically in an aluminum pan and heated from 30 to 400°C at a rate of 10°C/min under a stream of nitrogen (flow rate of 20 mL/min). For the thermogravimetric analysis (TGA), approximately 10 mg of the sample is weighed into aluminum pans and heated from 30 to 400°C at a heating rate of 10°C/min under nitrogen purge. The DSC thermogram indicates two thermal events: (i) an endothermic peak at 50°C possible due to desolvation or dehydration. This is further supported by the TGA thermogram which also indicates a weight loss exactly at the same temperature range, and (ii) the exothermic peak at 360°C probably due to the melting of the drug with decomposition.

### Powder X-Ray Diffraction Studies

The samples free acid (ON. 1210), the salt (ON.1210.Na) and the precipitated sample from filtered saturated ON.1210.Na solution kept at refrigerated condition are analyzed using Siemens powder X Ray diffractometer under ambient conditions. The scan range (2θ) is from 5° to 40° (Step scan, step size of 0.05°, with a dwell time of 1 second).

**Table 8: Peak Search Report for ON.1210 Acid**

| Peak Search Report (21 Peaks, Max P/N = 6.7) | | | | | | | |
|---|---|---|---|---|---|---|---|
| PEAK: 11-pts/Parabolic Filter, Threshold=3.0, Cutoff=0.1%, BG=3/1.0, Peak-Top=Summit | | | | | | | |
| **2-Theta** | **d(Å)** | **BG** | **Height** | **I%** | **Area** | **I%** | **FWHM** |
| 6.048 | 14.6011 | 20 | 102 | 52.9 | 559 | 48.2 | 0.234 |
| 10.07 | 8.777 | 9 | 12 | 6 | 57 | 4.9 | 0.196 |
| 10.341 | 8.5478 | 9 | 12 | 6 | 38 | 3.3 | 0.131 |
| 13.424 | 6.5905 | 8 | 3 | 1.8 | 7 | 0.6 | 0.1 |
| 14.046 | 6.2999 | 10 | 14 | 7.3 | -40 | -3.4 | 0.05 |
| 15.002 | 5.9008 | 7 | 25 | 13.1 | 89 | 7.7 | 0.15 |
| 15.75 | 5.6219 | 6 | 23 | 11.8 | 100 | 8.6 | 0.187 |
| 17.144 | 5.1681 | 14 | 181 | 94.2 | 896 | 77.2 | 0.198 |
| 17.495 | 5.0651 | 16 | 192 | 100 | 1160 | 100 | 0.256 |
| 18.251 | 4.8569 | 18 | 97 | 50.2 | 401 | 34.6 | 0.177 |
| 18.827 | 4.7096 | 14 | 13 | 6.7 | 150 | 12.9 | 0.468 |
| 19.147 | 4.6316 | 16 | 13 | 6.9 | 35 | 3 | 0.105 |
| 20.197 | 4.393 | 10 | 24 | 12.4 | 124 | 10.7 | 0.221 |
| 20.519 | 4.325 | 11 | 16 | 8.4 | 124 | 10.7 | 0.326 |
| 23.65 | 3.7589 | 11 | 22 | 11.7 | 115 | 9.9 | 0.217 |
| 28.24 | 3.1576 | 11 | 21 | 10.7 | 137 | 11.8 | 0.283 |
| 29.291 | 3.0466 | 10 | 17 | 9 | 40 | 3.4 | 0.098 |
| 30.563 | 2.9226 | 8 | 26 | 13.7 | 188 | 16.2 | 0.304 |
| 32.797 | 2.7285 | 8 | 9 | 4.8 | 23 | 2 | 0.1 |
| 35.9 | 2.4994 | 6 | 10 | 5.2 | 37 | 3.2 | 0.149 |
| 39.383 | 2.286 | 6 | 11 | 5.5 | 28 | 2.4 | 0.107 |

**Table 9: Peak Search Report for ON.1210 Na (13 Peaks, Max P/N = 10.6)**

| PEAK: 11-pts/Parabolic Filter, Threshold=3.0, Cutoff=0.1%, BG=3/1.0, Peak-Top=Summit | | | | | | | |
|---|---|---|---|---|---|---|---|
| **2-Theta** | **d(Å)** | **BG** | **Height** | **I%** | **Area** | **I%** | **FWHM** |
| 6.606 | 13.3698 | 14 | 73 | 16 | 204 | 10.1 | 0.119 |
| 7.366 | 11.9924 | 12 | 16 | 3.6 | 26 | 1.3 | 0.063 |
| 8.815 | 10.024 | 8 | 156 | 34.1 | 535 | 26.4 | 0.146 |
| 9.544 | 9.2595 | 9 | 22 | 4.7 | 54 | 2.7 | 0.106 |
| 17.762 | 4.9896 | 11 | 25 | 5.6 | 25 | 1.3 | 0.05 |
| 19.756 | 4.4902 | 8 | 20 | 4.4 | 224 | 11.1 | 0.478 |
| 23.647 | 3.7595 | 10 | 16 | 3.6 | 33 | 1.6 | 0.086 |
| 24.153 | 3.6817 | 8 | 22 | 4.8 | 59 | 2.9 | 0.114 |
| 28.968 | 3.0799 | 9 | 456 | 100 | .2025 | 100 | 0.189 |
| 31.074 | 2.8757 | 8 | 31 | 6.9 | 28 | 1.4 | 0.05 |
| 31.453 | 2.842 | 6 | 107 | 23.5 | 332 | 16.4 | 0.132 |
| 33.765 | 2.6524 | 6 | 16 | 3.5 | 67 | 3.3 | 0.178 |
| 39.002 | 2.3075 | 11 | 282 | 61.9 | 1481 | 73.2 | 0.223 |

Figure clearly shows that the sodium salt of ON.1210 is a crystalline material.

### Characterization of Precipitate

The sodium salt has a tendency to form a supersaturated solution in water, Upon storage, the solution starts showing some precipitate. The precipitate is isolated, dried and characterized by HPLC, DSC, TGA and XRPD.

The precipitate is dissolved in acetonitrile water mixture and analyzed by HPLC. The precipitate elutes at the same time as the standard of ON.1210.Na suggesting that the precipitate is of ON.1210.

The precipitate is further characterized by DSC. A thermogram of the precipitate does not show the transition around 50°C as is seen with the ON.1210.Na standard. However, it does show the same melting / decomposition around 360°C.

The precipitate is further characterized by XRPD. The diffractogram shows most of the peaks that are observed with ON.1210.Na except the one observed at 8.815.

**Table 10: Peak search report of precipitated sample**

| Peak Search Report (6 Peaks, Max P/N = 10.9) | | | | | | | |
|---|---|---|---|---|---|---|---|
| PEAK: 11-pts/Parabolic Filter, Threshold=3.0, Cutoff=0.1%, BG=3/1.0, Peak-Top=Summit | | | | | | | |
| **2-Theta** | **d(Å)** | **BG** | **Height** | **I%** | **Area** | **I%** | **FWHM** |
| 9.52 | 9.2823 | 7 | 44 | 9.2 | 159 | 9.1 | 0.153 |
| 16.749 | 5.2891 | 6 | 23 | 4.7 | 116 | 6.7 | 0.216 |
| 21.83 | 4.068 | 6 | 24 | 4.9 | 101 | 5.8 | 0.182 |
| 26.461 | 3.3656 | 4 | 10 | 2.1 | 42 | 2.4 | 0.168 |
| 28.957 | 3.081 | 6 | 481 | 100 | 1742 | 100 | 0.154 |
| 38.981 | 2.3087 | 5 | 168 | 35 | 812 | 46.6 | 0.205 |

In conclusion, this drug, e.g., ON.1210.Na, is surprisingly demonstrated to be quite stable in an aqueous environment at biological pH. Therefore, it can be formulated as an efficacious shelf stable parenteral formulation. We have further discovered that the aqueous solubility of the drug is low and can be enhanced by increased pH, cosolvents and, by formation of inclusion complex.

Related compounds include, but are not limited to (E)-4-fluorostyryl-4-chlorobenzylsulfone; (E)4-chlorostyryl-4-chlorobenzylsulfone; (E)-2-chloro-4-fluorostyryl-4-chlorobenzylsulfone; (E)-4-carboxystyryl-4-chlorobenzyl sulfone; (E)-4-fluorostyryl-2,4-dichlorobenzylsulfone; (E)-4-fluorostyryl-4-bromobenzylsulfone; (E)-4-chlorostyryl-4-bromobenzylsulfone; (E)-4-bromostyryl-4-chlorobenzylsulfone; (E)-4-fluorostyryl-4-trifluoromethylbenzylsulfone; (E)-4-fluorostyryl-3,4-dichlorobenzylsulfone; (E)-4-fluorostyryl-4-cyanobenzylsulfone; (E)-2,4-dichloro-4-chlorobenzylsulfone; and (E)-4-chlorostyryl-2,4-dichlorobenzylsulfone.

Related compounds include, but are not limited to (Z)-4-chlorostyryl-4-chlorobenzylsulfone; (Z)-4-chlorostyryl-4-fluorobenzylsulfone; (Z)-4-fluorostyryl-. 4-chlorobenzylsulfone; (Z)-4-bromostyryl-4-chlorobenzylsulfone; and (Z)-4-bromostyryl-4-fluorobenzylsulfone.

Related compounds are disclosed, contemplated, or exemplified in U.S. Patent No.6,656,973, including but not limited to synthesis examples 1-219.

### EXAMPLES

### EXAMPLE I

### Preparation of ON.1210.Na salt

4-Chlorobenzyl-4-carboxystyryl sulfone (ON 01210) (49 g; 0.145 mol) was taken in a one-liter conical flask and 500 ml of distilled water was added. Sodium hydroxide solution (16 ml: 10 M stock) (0.150 mol.) was added to the conical flask. The contents of the flask were then boiled with stirring till ON.01210 was completely dissolved. The solution was then cooled to room temperature and shining crystals separated were filtered through a fluted filter paper. The crystalline material was dried under vacuum to yield (48 g) (92% yield) of pure ON.1210.Na.

### EXAMPLE II

### Radioprotective Effects of α,β-Unsaturated Arylsulfones on Cultured Normal Cells

The radioprotective effects of the compounds in Table 11 below on cultured normal cells were evaluated as follows.

HFL-1 cells, which are normal diploid lung fibroblasts, were plated into 24 well dishes at a cell density of 3000 cells per 10 mm² in DMEM completed with 10% fetal bovine serum and antibiotics. The test compounds listed in Table 11 were added to the cells 24 hours later in select concentrations from 2.5 to 20 micromolar, inclusive, using DMSO as a solvent. Control cells were treated with DMSO alone. The cells were exposed to the test compound or DMSO for 24 hrs. The cells were then irradiated with either 10 Gy (gray) or 15 Gy of ionizing radiation (IR) using a J.L. Shepherd Mark I, Model 30-1 Irradiator equipped with ¹³⁷cesium as a source.

After irradiation, the medium on the test and control cells was removed and replaced with fresh growth medium without the test compounds or DMSO. The irradiated cells were incubated for 96 hours and duplicate wells were trypsinized and replated onto 100 mm² tissue culture dishes. The replated cells were grown under normal conditions with one change of fresh medium for 3 weeks. The number of colonies from each 100 mm² culture dish, which represents the number of surviving cells, was determined by staining the dishes as described below.

To visualize and count the colonies derived from the clonal outgrowth of individual radioprotected cells, the medium was removed and the plates were washed one time with room temperature phosphate buffered saline. The cells were stained with a 1:10 diluted Modified Giemsa staining solution (Sigma) for 20 minutes. The stain was removed, and the plates were washed with tap water. The plates were air dried, the number of colonies from each plate were counted and the average from duplicate plates was determined.

The results are presented in Table 11. A "+" indicates radioprotective activity of between 2- and 4.5-fold at the concentrations tested. Fold protection was determined by dividing the average number of colonies from the test plates by the average number of colonies on the control plates.

**Table 11: Radioprotection by α,β-Unsaturated Arylsulfones**

| Compound Number | Chemical Name | Activity |
|---|---|---|
| 1 | (E)-4-Fluorostyryl-4-chlorobenzylsulfone | + |
| 2 | (E)-2,4,6-Trimethoxystyryl-4-methoxybenzylsulfone | + |
| 3 | (E)-2-Methoxystyryl-4-nitrobenzylsulfone | - |
| 4 | (E)-2,3,4,5,6-Pentafluorostyryl-4-methoxybenzylsulfone | - |
| 5 | (E)-4-Fluorostyryl-4-trifluoromethylbenzylsulfone | + |
| 6 | (E)-4-Fluorostyryl-4-cyanobenzylsulfone | + |
| 7 | (Z)-4-Fluorostyryl-4-chlorobenzylsulfone | + |
| 8 | (E)-3-Furanethenyl-2,4-dichlorobenzylsulfone | + |
| 9 | (E)-4-Pyridylethenyl-4-chlorobenzylsulfone | - |
| 10 | (E)-4-Fluorostyryl-4-chlorophenylsulfone | + |
| 11 | (Z)-Styryl-(E)-2-methoxy-4-ethoxystyrylsulfone | + |
| 12 | (E)-4-Hydroxystyryl-4-chlorobenzylsulfone | - |
| 13 | (E)-4-Carboxystyryl-4-chlorobenzylsulfone | + |

### EXAMPLE III

### Protection of Mice from Radiation Toxicity by Pre-Treatment With (E)-4-Carboxystyryl-4-Chlorobenzylsulfone

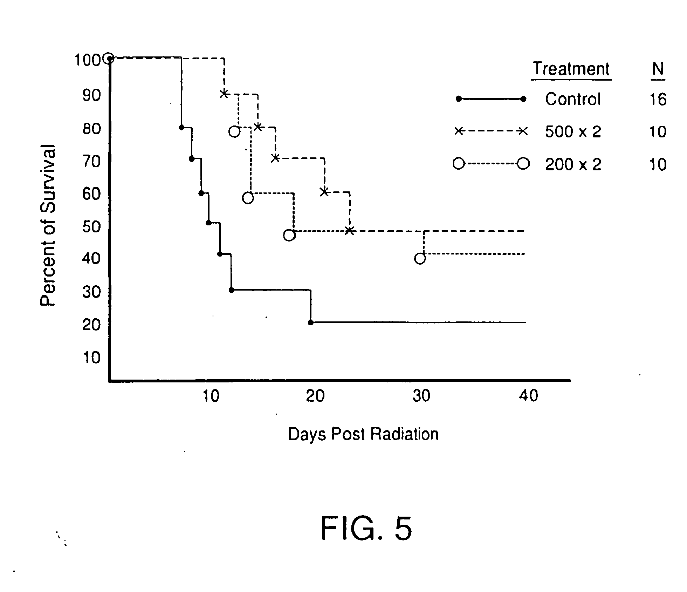

C57 black mice age 10-12 weeks (Taconic) were divided into two treatment groups of 10 mice each. One group, designated the "200x2" group, received intraperitoneal injections of 200 micrograms (E)-4-carboxystyryl-4-chlorobenzylsulfone dissolved in DMSO (a 10 mg/Kg dose, based on 20 g mice) 18 and 6 hours before irradiation with 8 Gy gamma radiation. The other group, designated "500x2," received intraperitoneal injections of 500 micrograms (E)-4-carboxystyryl-4-chlorobenzylsulfone dissolved in DMSO (a 25 mg/Kg dose, based on 20 g mice) 18 and 6 hours before irradiation with 8 Gy gamma radiation. A control group of 16 animals received 8 Gy gamma radiation alone. Mortality of control and experimental groups was assessed for 40 days after irradiation, and the results are shown in FIG.5.

By day 20 post-irradiation, the control mice exhibited a maximum mortality rate of 80%; the 8 Gy dose of gamma radiation is thus considered an LD₈₀ dose. By contrast, only about 50% of the "200x2" group and about 30% of the "500x2" mice were dead at day 20 after receiving the LD₈₀ radiation dose. By day 40, a maximum mortality rate of approximately 60% was reached in the "200x2" group, and a maximum mortality rate of approximately 50% was reached in the "500x2" group. These data show that radiation toxicity in mice is significantly reduced by pre-treatment with (E)-4-carboxystyryl-4-chlorobenzylsulfone.

### EXAMPLE IV

### Radioprotective Effect of (E)-4-Carboxystyryl-4-chlorobenzylsulfone in Mice When Given After Radiation Exposure

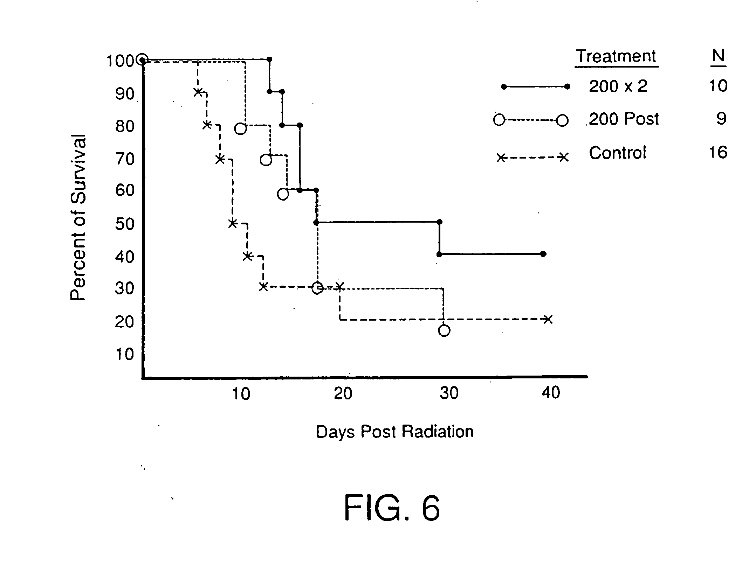

C57 B6/J mice age 10-12 weeks (Taconic) were divided into two treatment groups of 10 and 9 mice, respectively. One group, designated the "200x2" group, received intraperitoneal injections of 200 micrograms (E)-4-carboxystyryl-4-chlorobenzylsulfone dissolved in DMSO (a 10 mg/Kg dose, assuming 20 g mice) 18 and 6 hours before irradiation with 8 Gy gamma radiation. The other group, designated "200 Post," received an intraperitoneal injection of 200 micrograms (E)-4-carboxystyryl-4-chlorobenzylsulfone dissolved in DMSO (a 10 mg/Kg dose, based on 20 g mice) 15 minutes after irradiation with 8 Gy gamma radiation. A control group of 16 animals received 8 Gy gamma radiation alone. Mortality of control and experimental groups was assessed for 40 days after irradiation, and the results are shown in FIG.6.

FIG.6 shows that treatment of mice with (E)-4-carboxystyryl-4-chlorobenzylsulfone after irradiation resulted in significant delay in radiation-induced mortality as compared with the control animals. While the radioprotection afforded by post-irradiation treatment is not as great as seen with pre-irradiation treatment, (E)-4-carboxystyryl-4-chlorobenzylsulfone is nonetheless effective in mitigating the effects of radiation toxicity after the subject has received the radiation dose.

### EXAMPLE V

### Formulation of 25mg/ml ON.1210.Na ((E)4-Carboxystyryl-4-chlorobenzylsulfone sodium salt (C₁₆H₁₂ClNaO₄S))

12.114 g Tromethamine (0.1M) and 2.9224 g EDTA (0.01 M) are in 0.5 L deionized water. To that solution 500 ml of PEG 400 is added and stirred constantly till the pH is equilibrated. The pH of the final solution is adjusted using either HCl / NaOH to 7.8.

To the above vehicle enough ON01210 Na is added to obtain the final concentration of 25 mg/ml. The pH of the formulation is adjusted with either HCl or NaOH to obtain the final pH of 8.5.

### EXAMPLE VI

The formulation prepared in example V was monitored for its physical stability. The physical stability of the formulation was compared with a similar formulation prepared in water, and that prepared using phosphate buffer.5

(All formulations are 1:1 w/buffer: PEG400 (see, e.g., Example V))

**Table 12**

| **Date** | **Sample Type** | **pH** | **Observations** | **Additional Comments** |
|---|---|---|---|---|
| 01/16/07 | H₂O prepared | 8.50 | All samples in solution | Samples prepared, ½ autoclaved. 1 vial of each placed at room temp. and refrigerator |
| | 0.02 M Phosphate Buffer prepared | | | |
| | 0.02 M Tris/EDTA Buffer prepared | | | |
| 01/17/07 | H₂O-*room* (autoclaved) | 7.44 | Precipitated | All autoclaved samples ppt. |
| | H₂O*-refrig* (autoclaved) | 7.62 | | |
| | 0.02 M Phosphate-*room* (autoclaved) | 7.25 | | |
| | 0.02 M Phosphate-*refrig* (autoclaved) | 7.52 | | |
| | 0.02 M Tris/EDTA-*room* (autoclaved) | 7.35 | | |
| | 0.02 M Tris/EDTA-*refrig* (autoclaved) | 7.36 | | |
| 01/18/07 | H₂O-*room* | 6.94 | Precipitated | |
| | H₂O-*refrig* | 7.92 | | |
| | 0.02 M Phosphate-*room* | 7.78 | Remain in solution | |
| | 0.02 M Phosphate-*refrig* | 8.45 | | |
| | 0.02 M Tris/EDTA-*room* | 8.11 | | |
| | 0.02 M Tris/EDTA-*refrig* | 8.51 | | |
| 01/22/07 | 0.02 M Phosphate-*room* | 7.69 | Precipitated | 0.02 M Tris/EDTA room temp sample was split and ½ was readjusted to pH 8.5 |
| | 0.02 M Phosphate-*refrig* | 8.26 | | |
| | 0.02 M Tris/EDTA-*room* | 7.80 | Remain in solution | |
| | 0.02 M Tris/EDTA-*room* (pH adjusted) | 8.50 | | |
| 01/29/07 | 0.2 M Tris/EDTA prepared | 8.50 | All samples in solution | Study repeated w/ 0.2 M buffer strength |
| 01/30/07 | 0.02 M Tris/EDTA-*room* | 7.76 | All samples remain in solution | |
| | 0.02 M Tris/EDTA-*room* (pH adjusted) | 8.24 | | |
| | 0.02 M Tris/EDTA-*refrig* | 8.18 | | |
| | 0.2 M Tris/EDTA-*room* (autoclaved) | 8.52 | | |
| | 0.2 M Tris/EDTA-*room* | 8.53 | | |
| | 0.2 M Tris/EDTA-*refrig* (autoclaved) | 8.51 | | |
| | 0.2 M Tris/EDTA-*refrig* | 8.53 | | |
| 02/05/07 | 0.02 M Tris/EDTA-*room* | 7.72 | Precipitated | |
| | 0.02 M Tris/EDTA-*room* (pH adjusted) | 7.90 | Remain in solution | |
| | 0.02 M Tris/EDTA-*refrig* | 8.03 | | |
| | 0.2 M Tris/EDTA-*room* (autoclaved) | 8.37 | | |
| | 0.2 M Tris/EDTA-*room* | 8.37 | | |
| | 0.2 M Tris/EDTA-*refrig* (autoclaved) | 8.83 | | Refrigerated samples, pH increased |
| | 0.2 M Tris/EDTA-*refrig* | 8.64 | | |
| 02/15/07 | 0.02 M Tris/EDTA-*room* (pH·adjusted) | 7.98 | Remain in solution | Again the refrigerated samples showed an increase in pH |
| | 0.02 M Tris/EDTA-*refrig* | 7.96 | | |
| | 0.2 M Tris/EDTA-*room* (autoclaved) | 8.62 | | |
| | 0.2 M Tris/EDTA-*room* | 8.58 | | |
| | 0.2 M Tris/EDTA-*refrig* (autoclaved) | 8.80 | Few particles ppt | |
| | 0.2 M Tris/EDTA-*refrig* | 8.89 | Precipitated | |
| 02/27/07 | 0.02 M Tris/EDTA-*room* (pH adjusted) | 7.80 | Remain in solution | |
| | 0.02 M Tris/EDTA-*refrig* | 8.07 | | |
| | 0.2 M Tris/EDTA-*room* (autoclaved) | 8.66 | | |
| | 0.2 M Tris/EDTA-*room* | 8.70 | | |
| 03/21/07 | 0.02 M Tris/EDTA-*room* (pH adjusted) | 7.85 | Few particles ppt | |
| | 0.02 M Tris/EDTA-*refrig* | 8.14 | Remain in solution | |
| | 0.2 M Tris/EDTA-*room* (autoclaved) | 8.68 | | |
| | 0.2 M Tris/EDTA-*room* | 8.74 | | |
| 03/30/07 | 0.02 M Tris/EDTA-*refrig* | 7.82 | Remain in solution | |
| | 0.2 M Tris/EDTA-*room* (autoclaved) | 8.51 | | |
| | 0.2 M Tris/EDTA-*room* | 8.54 | | |
| 05/03/07 | 0.02 M Tris/EDTA-*refrig* | 8.03 | Remain in solution | |
| | 0.2 M Tris/EDTA-*room* (autoclaved) | 8.43 | | |
| | 0.2 M Tris/EDTA-*room* | 8.46 | | |
| 07/16/07 | 0.02 M Tris/EDTA-*refrig* | | Precipitated | |
| | 0.2 M Tris/EDTA-*room* (autoclaved) | | Remain in solution | |
| | 0.2 M Tris/EDTA-*room* | | Remain in Solution | |

### EXAMPLE VII (not part of the claims)

### Example Suspension Formulation

### 1L 50mg/mL ON.1210.Na suspension

13.609g KH2PO4
8.766g NaCl
10mL Tween 80
50g ON1210.Na
and qs to 1L with D.I. water.

The suspension is then mixed by stirring and pH is adjusted to 8.2 and stirred overnight. The pH is checked the following day (it decreases overnight) and is readjusted to ph8.2. Continue this process untill the pH stabilizes.

Various modifications and variations of the described subject matter will be apparent to those skilled in the art. Although the invention has been described in connection with specific embodiments, it should be understood that the invention as claimed should not be unduly limited to these embodiments. Indeed, various modifications for carrying out the invention are obvious to those skilled in the art and are intended to be within the scope of the following claims.

## Claims

1. An aqueous pharmaceutical solution composition suitable for administration for reducing toxic effects of ionizing radiation in a subject, comprising 20mg/ml to 100mg/ml of sodium salt of (E)-4-carboxystyryl-4-chlorobenzylsulfone (ON 01210. Na); Tris-EDTA buffer composed of Tris within the range of 0.1M to 0.3M and EDTA within the range of 0.01M to 0.03M; PEG 400 within the range of 40% w/v to 60% w/v; and, wherein the composition has a pH within the range of 8.3 to 8.7.

2. The composition of claim 1 which comprises 20mg/ml to 60mg/ml of sodium salt of (E)-4-carboxystyryl-4-chlorobenzylsulfone (ON 01210. Na); Tris-EDTA buffer composed of Tris within the range of 0.15M to 0.25M and EDTA within the range of 0.015M to 0.025M; PEG 400 within the range of 45% w/v to 55% w/v; and, wherein the composition has a pH within the range of 8.4 to 8.6.

3. The composition of claim 1 which comprises 25mg/ml to 50mg/ml of sodium salt of (E)-4-carboxystyryl-4-chlorobenzylsulfone (ON 01210. Na); Tris-EDTA buffer composed of Tris of about 0.2M and EDTA of about 0.02M; about 50% w/v PEG 400; and, wherein the composition has a pH within the range of 8.4 to 8.6.

## Patentansprüche

1. Wässrige pharmazeutische Lösungszusammensetzung, die zur Verabreichung zur Verringerung der toxischen Wirkungen von ionisierender Strahlung bei einem Patienten geeignet ist, umfassend 20 mg/ml bis 100 mg/ml Natriumsalz von (E)-4-Carboxystyryl-4-chlorbenzylsulfon (ON 01210. Na); Tris-EDTA-Puffer aus Tris im Bereich von 0,1 M bis 0,3 M und EDTA im Bereich von 0,01 M bis 0,03 M; PEG 400 im Bereich von 40% w/v bis 60% w/v; und wobei die 2zusammensetzung einen pH-Wert im Bereich von 8,3 bis 8,7 aufweist.

2. 2zusammensetzung nach Anspruch 1, die 20 mg/ml bis 60 mg/ml Natriumsalz von (E)-4-Carboxystyryl-4-chlorbenzylsulfon (ON 01210. Na); Tris-EDTA-Puffer aus Tris im Bereich von 0,15 M bis 0,25 M und EDTA im Bereich von 0,015 M bis 0,025 M; PEG 400 im Bereich von 45% w/v bis 55% w/v umfasst; und wobei die Zusammensetzung einen pH-Wert im Bereich von 8,4 bis 8,6 aufweist.

3. Zusammensetzung nach Anspruch 1, die 25 mg/ml bis 50 mg/ml Natriumsalz von (E)-4-Carboxystyryl-4-chlorbenzylsulfon (ON 01210. Na); Tris-EDTA-Puffer aus Tris im Bereich von etwa 0,2 M und EDTA im Bereich von etwa 0,02 M; etwa 50% w/v PEG 400 umfasst; und wobei die Zusammensetzung einen pH-Wert im Bereich von 8,4 bis 8,6 aufweist.

## Revendications

1. Composition pour solution pharmaceutique aqueuse adaptée à une administration visant à réduire les effets toxiques des rayonnements ionisants chez un sujet, comprenant entre 20 mg/ml et 100 mg/ml de sel de sodium de (E)-4-carboxystyryl-4-chlorobenzylsulfone (ON 01210 . Na) ; un tampon Tris-EDTA composé de Tris dans l'intervalle compris entre 0,1M et 0,3M et d'EDTA dans l'intervalle compris entre 0,01M et 0,03M ; du PEG 400 dans l'intervalle compris entre 40 % en masse par volume et 60 % en masse par volume ; et où la composition présente un pH dans l'intervalle compris entre 8,3 et 8,7.

2. Composition conforme à la revendication 1 qui comprend entre 20 mg/ml et 60 mg/ml de sel de sodium de (E)-4-carboxystyryl-4-chlorobenzylsulfone (ON 01210 · Na) ; un tampon Tris-EDTA composé de Tris dans l'intervalle compris entre 0,15M et 0,25M et d'EDTA dans l'intervalle compris entre 0,015M et 0,025M ; du PEG 400 dans l'intervalle compris entre 45 % en masse par volume et 55 % en masse par volume ; et où la composition présente un pH dans l'intervalle compris entre 8,4 et 8,6.

3. Composition conforme à la revendication 1 qui comprend entre 25 mg/ml et 50 mg/ml de sel de sodium de (E)-4-carboxystyryl-4-chlorobenzylsulfone (ON 01210 · Na) ; un tampon Tris-EDTA composé de Tris à environ 0,2M et d'EDTA à environ 0,02M ; d'environ 50 % en masse par volume de PEG 400 ; et où la composition présente un pH compris entre 8,4 et 8,6.
